# EUROPEAN PATENT SPECIFICATION

(11) **EP 0 662 087 B1**
(45) Date of publication and mention of the grant of the patent: **01.10.2003**
(21) Application number: 93914393.9
(22) Date of filing: 04.06.1993
(51) Int. Cl.: C12N 15/12, C07K 14/475, C07K 14/81

(54) **METHOD FOR PURIFYING RETINAL PIGMENTED EPITHELIUM DERIVED NEUROTROPHIC FACTOR**
VERFAHREN ZUR REINIGUNG EINES NEUROTROPHEN FAKTORS AUS PIGMENTIERTEM RETINALEN EPITHELGEWEBE
PROCEDE POUR LA PURIFICATION D'UN FACTEUR NEUROTROPHIQUE DERIVE DE L'EPITHELIUM RETINIEN PIGMENTE

(30) Priority: 04.06.1992 US 894215
(43) Date of publication of application: 12.07.1995
(73) Proprietor: THE UNIVERSITY OF SOUTHERN CALIFORNIA, Los Angeles, California 90089 (US)
(72) Inventor: JOHNSON, Lincoln, V., Pasadena, CA 91105 (US); TOMBRAN-TINK, Joyce, Silverspring, MD 20910 (US)
(74) Representative: Barth, Renate, Dr.
(86) International application number: US9305358
(87) International publication number: WO93024529

(56) References cited:
- WO-A-91/03568
- EXPERIMENTAL EYE RESEARCH, vol. 53, 1991, pages 411-414, XP000569293 J. TOMBRAN-TINK ET AL: "PEDF: A pigment epithelium-derived factor with potent neuronal differentiative activity"
- Proc. Natl. Acad. Sci. USA, Volume 90, issued February 1992, F.R. STEELE, "Pigment Epithelium-Derived Factor: Neurotrophic Activity and Identification as a Member of the Serine Protease Inhibitor Gene Family", pages 1526-1530, see entire document, especially figure 2.
- Developmental Biology, Volume 148, issued 1991, C.M. PARK et al., "Induction of Retinal Regeneration in Vivo by Growth Factors", pages 322-333.
- TOMBRAN-TINK J. ET AL INVESTIGATIVE OPHTHALMOLOGY & VISUAL SCIENCE vol. 32, no. 4, 1991, page 755

## Description

### Field of the Invention

This invention relates to the purification of a retinal pigmented epithelium derived neurotrophic factor (PEDNF).

### Background of the Invention

Many types of neurons depend upon the availability of special regulatory molecules, known as neurotrophic factors, for their survival and well-being. The best characterized of the neurotrophic factors is nerve growth factor (NGF). NGF regulates the survival and specialized function of sympathetic and dorsal root ganglion neurons in the peripheral nervous system and of some cholinergic neurons in the central nervous system. Trophic factors, which act on other neurons, have also been identified, and two such factors, ciliary neurotrophic factor (CNTF) and brain-derived neurotrophic factor (BDNF) have been purified. Moreover, it has recently been shown that some growth factors, such as fibroblast growth factor (FGF) and epidermal growth factor (EGF), which initially were identified based on their mitogenic effects upon cells, also function as survival-promoting agents for some neurons. Post-synaptic target cells and satellite cells, such as glia cells, appear to be major sources of neurotrophic factors.

It has been proposed that the survival of retinal photoreceptor cells may also be regulated by specific neurotrophic factors. Evidence supporting this concept includes the observation that photoreceptors undergo developmental neuronal death in some species, a phenomenon which is generally considered to reflect the limited availability of neurotrophic factors. Photo-receptor development, as well as maintenance of normal function, has also been shown to require interactions with the retinal pigment epithelium (RPE), suggesting that RPE-derived molecules or factors could be necessary for photoreceptor function and survival.

The RPE develops in advance of and lies adjacent to the neural retina. A closed compartment between the two cell layers contains the interphotoreceptor matrix, and many soluble secretory products of RPE and neural retina cells are contained in the interphotoreceptor matrix. Nutrients, metabolites or trophic factors exchanged between the RPE and neural retina, must pass through the interphotoreceptor matrix. RPE cells, for example, are thought to synthesize and secrete a photoreceptor survival-promoting factor (PSPA).

Cultured RPE cells synthesize a number of well known trophic factors, including platelet derived growth factor (PDGF), FGF, transforming growth factor-α (TGF-α), and transforming growth factor-β (TGF-β). It is possible that these or other unknown factors derived from RPE could influence the development of the neural retina.

Tombran - Tink et al. (Exp. Eye Res. (1991) 53, pages 411 - 414) disclose the purification of a RPE derived factor with potent neuronal differentiative activity.

The neural-derived RPE forms a monolayer of cells interposed between the neural retina and circulating blood within the choroid. In this strategic location, the RPE forms a part of the blood-retina barrier, performs functions essential to retinal integrity and functions, and plays important roles in vascular, inflammatory, degenerative, and dystrophic diseases of the retina and choroid. The functions of the RPE in relation to the visual process are several-fold and include light-dark adaption, phagocytosis of shed rod outer segment membrane and nutrition. On the other hand, the close interdependence of the RPE and the neural retina during normal development have been known for a long time, but functionally are not well understood, although it is known that the RPE is important for retinal regeneration. It has been consistently observed that loss of contact of the neural retina with the RPE of many vertebrates (retinal detachment) results in degeneration of the retina. As a side effect of the retinal detachment, strong cell proliferation, originating from the RPE which underlies the spots of detachment, has often been observed.

Thus, identification of hypothetical survival-promoting factors for photoreceptor cells would potentially be of great importance for the treatment of pathological conditions which result in blindness due to photoreceptor degeneration of unknown etiology. While these types of selective photoreceptor degenerations could be due to a variety of different mechanisms, analogies with neuronal degenerations in other regions of the nervous system suggest the possible involvement of a neurotrophic activity in the retina.

### Summary of the Invention

The present invention relates to a method for purifying a retinal pigmented epithelium neurotrophic factor. The purification procedure comprises providing an impure protein fraction containing retinal pigmented epithelium derived neurotrophic factor, precipitating contaminating proteins from the impure protein fraction containing PEDNF by bringing the impure protein fraction containing PEDNF to 50% to 60% saturation with ammonium sulfate to provide a 50% to 60% ammonium sulfate protein fraction, and/or precipitating PEDNF from the impure protein fraction containing PEDNF by bringing the impure protein fraction containing PEDNF to 70% to 80% saturation with ammonium sulfate to provide a 70% to 80% ammonium sulfate protein fraction and applying the impure protein fraction containing retinal pigmented epithelium derived neurotrophic factor to a cation-exchange chromatography medium. The cation-exchange chromatography medium is then washed to elute any unbound proteins and the retinal pigmented epithelium derived neurotrophic factor is eluted from the cation-exchange chromatograph medium and collected.

Further described is a recombinant DNA molecule comprising a gene encoding a retinal pigmented epithelium derived neurotrophic factor having the DNA sequence or the amino acid sequence in SEQ ID NO 1 and a non-human organism transformed with a recombinant DNA molecule comprising a retinal pigmented epithelium derived neurotrophic factor gene having a DNA sequence identified in SEQ ID NO 1.

PEDNF purified according to the present invention can be used in a method of treating tumors, ocular diseases and conditions resulting from the activity of serine proteases which comprises administering PEDNF.

### Brief Description of the Drawings

Features and advantages of the invention will be more apparent from a reading of the claims and of the detailed description of the invention in conjunction with the drawings described below.

Fig. 1 is a Coomassie blue stained SDS polyacrylamide gel showing the location of the PEDNF protein doublet unique to RPE-conditioned medium (RPE-CM).

Fig. 2 is a differential interference contrast micrograph showing cell cultures 8-day post-attachment stimulated for 7 days in serum-free medium containing 2 µg/ml of electroeluted PEDNF (panel A) or serum-free control medium (panel B).

Fig. 3 is a silver-stained, two-dimensional gel. Molecular weights are indicated at the left, and pH extremes are indicated at the top.

### Detailed Description

Cells of the retinal pigmented epithelium are closely associated with differentiating retinoblasts in vivo and contribute to an environment essential to their development and normal function, both in vivo and in vitro. Retinoblastoma cells exhibit a multipotential differentiative nature paralleling that of their precursor, the primitive retinoblast, as evidenced by the fact that agents, such as laminin, sodium butyrate and dibutyryl cAMP, can induce the expression of neuronal, glial, and pigmented epithelial characteristics in vitro.

A human retinoblastoma cultured cell line, Y79 cells, when exposed to RPE-conditioned medium (RPE-CM), exhibits a high degree of neuronal-like differentiation. The differentiation is observed in both the morpho- logical and biochemical characteristics of the cells. The exposed cells extend arborizing neuritic processes from their cell bodies and express elevated levels of neuron-specific enolase (NSE) and neurofilament proteins.

RPE-secreted proteins have been fractionated from RPE-CM, and a protein doublet, with an apparent molecular weight of about 50,000 to about 55,000, that is unique to RPE-CM has been identified. This pigment epithelium derived neurotrophic factor (PEDNF), which is a major secretory product of human fetal RPE cells, has been isolated and shown to have neurotrophic effects on Y79 retinoblastoma cells.

PEDNF has uses in the treatment of retinal diseases including, but not limited to, retinoblastoma and other ocular tumors, retinitis pigmentosa, various forms of retinal detachment, macular degeneration, diabetic retinopathy, and other inherited and age-related pathologies of retinal cells.

In the case of retinal tumors, PEDNF induces the tumor cells to display biochemical and phenotypic characteristics of mature neuronal cells. Such changes are identified by a cessation or reduction in the rate of cell division, which leads to tumor regression or a slowing in the rate of tumor growth. In the case of non-tumorous retinal diseases, the neurotrophic properties of PEDNF enhance survival and well-being of photoreceptor or other retinal cells, prolonging their functional life span and delaying the rate of the onset of impaired vision and ultimate blindness. In the case of retinal detachment, PEDNF prolongs the life span of photoreceptor cells sufficiently to allow standard reattachment procedures to be effective in re-estab-lishing the retina-RPE interface, thereby restoring normal vision.

### 1. Preparation of an Impure Protein Fraction Containing Retinal Pigmented Epithelium Derived Neurotrophic Factor

Retinal pigmented epithelium derived neurotrophic factor (PEDNF) may be isolated from any tissue or cell producing PEDNF.

Naturally-occurring cells that produce PEDNF are retinal pigmented epithelium cells. Such cells may be grown in culture to secrete PEDNF into the medium in which they are growing. The medium may be harvested periodically, and the PEDNF isolated from the media. Suitable cell cultures may be established from retinal pigmented epithelium cells derived from humans, monkeys, and other primates or other animals, such as chickens, mice, rats, cows and pigs.

PEDNF may also be isolated by extraction of the interphotoreceptor matrix (IPM) or from the retina of humans, monkeys, and other primates or other animals, such as chickens, mice, rats, cows and pigs.

Alternatively PEDNF may be derived from sources in which it is not naturally-occurring, such as non-human organisms transfected with a recombinant DNA molecule constructed to result in the expression of PEDNF in the host cells chosen for the expression of the gene.

### A. Culturing of Human Retinal Pigmented Epithelium (RPE) Cells

Cultures of RPE cells are established by harvesting post-mortem eyes by aseptically opening the eyes and removing the vitreous body and retina. The exposed RPE is washed with a buffered solution such as modified Earle's balanced salt solution (MEBS: 115.5 mM NaCI, 3.5 mM KCI, 1 mM NaH₂PO₄, 0.5 mM CaCl₂, 0.27 mM MgCl₂, 0.37 MgSO₄, 15 mM HEPES, 14 mM NaHCO₃, 12 mM glucose, pH 7.2). RPE cells are scraped from Bruch's membrane or are dislodged by a stream of fluid such as MEBS or cell culture medium, applied using a Pasteur or similar pipette. This step may be preceded by exposure to proteolytic or other enzyme(s), such as Dispase (Boehringer-Mannheim, Indianapolis, IN, Catalog #295 825). Alternatively, RPE cells may be isolated by detaching sections of sclera from an intact eye to expose choroidal tissue and treating this choroidal surface with proteolytic or other enzymes such as Dispase prior to removal of the vitreous and retina and dislodging RPE cells by the spraying of cell culture medium as described by Pfeffer. Tissue fragments are transferred to tissue culture dishes in a medium such as "low Ca⁺⁺" or "high Ca⁺⁺" complete RPE-47 medium, as described by Pfeffer, or Eagles's minimal essential medium (MEM, supplied by GIBCO of Grand Island, NY) supplemented with about 0.5% to about 20%, by volume, fetal calf serum. At concentrations below about 0.5% fetal calf serum, the serum concentrations are too low to effectively support the growth of the cells. At concentrations above about 20% serum, no additional benefit, with respect to cell growth, is conferred on the cells.

The medium may also be supplemented with anti- biotics and/or fungicides to prevent the growth of bacteria and/or fungi in the cultures. Antibiotics and fungicides suitable for use in the present invention are about 1,000 units/ml penicillin, about 100 µg/ml streptomycin, about 0.25 µg amphotericin, and about 50 µg/ml gentamicin, or other suitable such agents known in the art. These antibiotics may be used individually or in combination, as desired.

The cells are incubated at about 37°C in an atmosphere of about 5% carbon dioxide. Retinal pigmented epithelium (RPE) cells attach to the surface of the dishes, proliferate, and eventually form confluent monolayers.

When the cells have grown to confluence, about 3-7 days from the initial culturing of the cells, they are harvested, for example, by trypsinizing the cell monolayer, or by other methods known to those skilled in the art, and resuspending the cells in a medium such as MEM, supplied by GIBCO, supplemented with about 5% to about 20% fetal calf serum. A portion of the cells are reseeded into sterile tissue culture flasks, after which time the cells are again grown in an atmosphere of about 5% CO₂ at about 37°C.

Alternatively, RPE cells may be isolated by removing the cornea, the 2 mm scleral ring, and the vitreous and neural retina from the eyes. The eyes are washed with calcium and magnesium-free Hanks Balanced Salt Solution (HBSS: 1.3 mM CaCl₂, 5 mM KCI, 0.3 mM KH₂PO₄, 0.5 mM MgCl₂, 0.4 mM MgSO₄, 138 mM NaCI, 4 mM NaHCO₃, 0.3 mM Na₂HPO₄, 5.6 mM D-glucose and 0.03 mM Phenol Red), supplied by GIBCO/BRL of Gaithersburg, MD. The eye cup is filled with a solution comprising about 0.1 %, by weight, trypsin, about 0.1%, by weight, hyaluronidase in calcium and magnesium-free Hanks balanced salt solution, and the eye is incubated at about 37°C for about 15 to about 30 min.

The loose RPE cells are collected by gentle aspiration, and the procedure is repeated until the RPE cells are released. The trypsin is inactivated by adding about 5% to about 20% fetal calf serum to the cell sample. The cells are collected by centrifugation at about 1,200 rpm for about 7 min.

The RPE cells are then plated onto sterile tissue culture plates at a density of about 1 x 10⁵ cells for a 35 mm plate. (Proportionally more or less cells are plated if larger or smaller plates or containers are used.) The cells are grown in DulBecco's Modified Eagles Medium (DMEM), supplied by GIBCO, or other suitable medium. The medium may be supplemented with an equal volume of HAM's F12 medium (supplied by GIBCO), about 1 mM sodium pyruvate, about 0.625 mM Hepes, about 6 mM L-glutamine, about 1 % non-essential amino acids, about 5 µg/ml insulin, about 5 µg/ml transferrin, about 5 ng/ml selenium, antibiotics as described above, and about 0.5% to about 20% fetal calf serum, as described above. The insulin, transferrin, and selenium are supplied by Collaborative Research of Lexington, MA. Other reagents suitable for use in the present invention, unless otherwise specified, are supplied by Sigma Chemical Co. of St Louis, MO.

When the cells have grown to confluence (about 3-7 days from the initial culturing of the cells), they are harvested, for example, by trypsinizing the cell monolayer or by other methods known to those skilled in the art, and resuspending the cells in a medium such as MEM supplemented with about 0.5% to about 20% fetal calf serum. A portion of the cells are reseeded into sterile tissue culture flasks, after which time the cells are again grown in an atmosphere of about 5% CO₂ at about 37 °C.

While only two methods for the culturing of RPE cells are described, other suitable methods for culturing RPE cells are known in the art. Such methods are also suitable for use in the practice of the present invention.

### B. Preparation of Retinal Pigmented Epithelium Conditioned Medium (RPE-CM)

PEDNF is a secreted protein, and RPE cells secrete PEDNF into the medium in which they are grown. There-fore, a convenient method of producing PEDNF is to grow RPE cells in culture and to periodically harvest the media from the cultures.

A method suitable for use in the present invention for isolating PEDNF from medium is to allow RPE cells to grow to confluence in a medium such as DMEM supplemented with about 1 mM sodium pyruvate, about 0.625 mM Hepes, about 6 mM L-glutamine, about 1 % non-essential amino acids, about 5 µg/ml insulin, about 5 µg/ml transferrin, about 5 ng/ml selenium, antibiotics and/or fungicides as described above, and about 0.5% to about 20% fetal calf serum, as described above. The confluent cultures of RPE cells are washed extensively with Hank's balanced salt solution, or other suitable wash solutions, to remove serum proteins derived from the fetal calf serum present in the media which is used to culture the RPE cells. About 1 ml, per cm² of cell surface area, of serum-free medium such as DMEM supplemented with about 1 mM sodium pyruvate, about 0.625 mM Hepes, about 6 mM L-glutamine about 1% non-essential amino acids, about 5 µg/ml insulin, about 5 µg/ml transferrin, about 5 ng/ml selenium, and antibiotics and/or fungicides as described above, is added to the cultures, and they are incubated in an atmosphere of about 5% CO₂ at about 37°C for about 1 to about 10 days. Alternatively, PEDNF can be isolated from serum-containing medium such as DMEM supplemented with about I mM sodium pyruvate, about 0.625 mM Hepes, about 6 mM 1-glutamine, about 1% non-essential amino acids, about 5 µg/ml insulin, about 5 µg/ml transferrin, about 5 ng/ml selenium, antiohotics and/or fungicides as described above, and about 0.5% to about 20% fetal calf serum as described above.

The medium is then collected by pouring the medium into plastic centrifuge tubes, and the medium is centrifuged at about 3,000 rpm for about 10 min. to remove any free cells and other particulate matter from the medium, and filtered to provide an impure PEDNF protein solution. The medium may be used directly for the purification or testing of PEDNF or it may be stored at -20°C until required.

### C. Isolation of PEDNF from Tissue Samples

PEDNF may be isolated directly from eyes by opening the eyes and removing the vitreous body and retina. The retinal pigmented epithelium is scraped off the Bruch's membrane using a disposable cell scraper. Tissue fragments are transferred to a Teflon or glass homogenizer in 10 mM phosphate-buffered saline (PBS) and homogenized to break the cells. The solution is then centrifuged at about 10,000 rpm for about 10 min. and filtered to remove cell debris. The supernatant, which contains PEDNF, is collected to provide an impure PEDNF protein solution. The medium may be used directly for the purification or testing of PEDNF or it may be stored at -20°C until required.

### D. Isolation of PEDNF from Recombinant Cells

PEDNF may also be isolated from recombinant cells which have been constructed to express the PEDNF gene. The PEDNF may be expressed as an intracellular or an extracellular protein.

Intracellular PEDNF is isolated by homogenizing the cells in a Dounce or other suitable homogenizer in a buffer, such as PBS, that may contain detergents or other solubilizing agents such as urea or guanidine hydrochlorid, and centrifuging at about 10,000 rpm for about 20 min. to remove cellular debris, to provide an impure PEDNF protein fraction.

Extracellular PEDNF is isolated by collecting the medium in which cells expressing PEDNF are grown. This is most conveniently performed in a continuous centrifu-gation process, such as with a Sharples centrifuge. The supernatant is collected to provide an impure PEDNF protein fraction. The medium may be used directly for the purification or testing of PEDNF or it may be stored at -20°C until required.

### 2. Purification of PEDNF

### A. Small Scale Purification of PEDNF

### i. Ammonium Sulfate Precipitation

An impure PEDNF protein fraction may be partially purified by ammonium sulfate precipitation to provide an ammonium sulfate purified PEDNF protein fraction. Percent ammonium sulfate refers to % saturation of ammonium sulfate at 20°C and is based on a 100% saturation of 767 g/l.

An impure PEDNF protein fraction is brought to about 50% saturation with ammonium sulfate by the addition of about 313 g of solid ammonium sulfate per liter of impure protein fraction at about 20°C. The ammonium sulfate is preferably added slowly to the impure protein fraction while the solution is stirred, such as with a stir bar on a mechanical stirrer. The ammonium sulfate is added slowly to prevent localized high concentrations of ammonium sulfate that may result in rapid precipitation and denaturation of proteins present in the impure protein fraction. After all the ammonium sulfate is added, the about-50% ammonium sulfate solution is stirred for about 30 min. at about 20°C. The about-50% ammonium sulfate solution is then centrifuged at about 10,000 g, at about 20°C for about 20 min. The supernatant is collected for further processing. Alternatively, the about-50% ammonium sulfate solution may be filtered through filter paper such as Whatman #1, to remove the precipitate. In this case, the filtrate is collected for further processing.

The about-50% ammonium sulfate solution is then brought to about 70% saturation by the addition of about 137 g/l of ammonium sulfate. The ammonium sulfate is added slowly, and after all the ammonium sulfate is added, the about-70% ammonium sulfate solution is stirred for about 30 min. at about 20°C. The about-70% ammonium sulfate solution is centrifuged or filtered, as described above, and the precipitate is collected.

The ammonium sulfate precipitate is then redissolved in a buffer such as about 10 mM phosphate, pH 7, to form a 50-70% ammonium sulfate fraction. The solution is then diafiltered using an Amicon Diaflo ultrafiltration unit, or dialyzed against a buffer such as about 10 mM phosphate, pH 7, to remove the residual ammonium sulfate from the redissolved 50%-70% ammonium sulfate fraction.

### ii. Purification of PEDNF by SDS Gel Electrophoresis

A small-scale isolation of PEDNF is conducted by SDS-polyacrylamide slab gels. Such polyacrylamide gels are well known in the art, and the preparation of such gels has been described by Weber and Osborn, J. Biol. Chem., 244, 4406 (1969), as modified by Laemmli, Nature. 277, 680 (1970).

Samples of an impure PEDNF protein fraction or ammonium sulfate purified PEDNF protein fraction are dialyzed against a buffer such as about 10 mM sodium phosphate buffer, pH 7.5, lyophilized and resuspended in 62.5 mM Tris, pH 6.8, 2% SDS, 10% glycerol, 0.001% bromophenol blue, and 0.1 M 2-mercaptoethanol, wherein the bromophenol blue is a migration marker. Additional samples to be loaded on the gel include molecular-weight standards such as phosphorylase B, bovine serum albumin, ovalbumin, carbonic anhydrase, soybean trypsin inhibitor and lysozyme (such as supplied by Bio-Rad, catalog #161-0304), and controls as may be necessary. In cases where conditioned media are used as samples, media which have not been exposed to RPE cells (unconditioned media) are included as controls.

The samples are then incubated in a boiling-water bath for about 5 min. and loaded onto SDS-polyacrylamide slab gels. The markers and unconditioned media are loaded into wells on the outside of the gel, and the impure PEDNF protein fractions or the ammonium sulfate purified PEDNF protein fraction are loaded on the remaining inside wells. The samples are then subjected to electrophoresis. The electrophoresis is continued until the bromphenol blue marker has reached the bottom of the gel. At the completion of electrophoresis, strips from each outside edge of the gels, which included the markers and a lane each of an impure PEDNF protein fraction and an unconditioned media control, are stained with Coomassie blue. The stained protein bands which develop on the stained strips are aligned with the unstained portion of the gel to locate the position of the proteins present in the impure PEDNF protein fraction or the ammonium sulfate purified PEDNF protein fraction but absent from control media.

A PEDNF protein doublet, with an apparent molecular weight of about 50,000 to about 55,000, unique to the impure PEDNF protein fraction, is excised and the proteins electroeluted, by methods known in the art, from the unstained portion of the gel. The eluant is centrifuged to remove gel fragments and the supernatant dialyzed against 10 mM phosphate-buffered saline (145 mM NaCI, 8.1 mM Na₂HPO₄, and 1.9 mM NAH₂PO₄.H₂O) to provide an SDS-polyacrylamide purified PEDNF protein fraction.

### iii. Purification of PEDNF by Cation-Exchange HPLC

An impure PEDNF protein fraction or an ammonium sulfate purified PEDNF protein fraction is dialyzed against water or a buffer such as about 10 mM phosphate buffer, pH 7.2, or other suitable buffer, to remove media and salts from the impure protein sample. PEDNF may be purified by cation-exchange HPLC using a column chromatography medium, such as that supplied under the trade name "Brownlee Aquapore CX-300" by Western Analytical, Temecula, CA, packed into a column, such as a 4.6 x 30 mm column, or other suitable cation-exchange HPLC chromatography medium.

The chromatography medium is equilibrated with a buffer such as about 10 mM phosphate, pH 7.2. The dialyzed impure PEDNF protein fraction or ammonium sulfate purified PEDNF protein fraction is loaded onto the chromatography medium, and the chromatography medium with PEDNF bound to it is washed with a buffer such as about 10 mM phosphate, pH 7.2, until all unbound proteins are washed from the chromatography medium. PEDNF is eluted from the chromatograph medium with a linear salt gradient from about 0.0 to about 0.5 M NaCI PEDNF elutes as a single peak with a NaCI concentration of about 0.25 M.

The eluted PEDNF is concentrated by lyophilization and resolubilized in water or a buffer such as about 10 mM phosphate buffer, pH 7.2, or other suitable buffer, to form a cation-exchange HPLC purified PEDNF protein fraction.

### iv. Purification of PEDNF by Reverse-Phase HPLC

An impure PEDNF protein fraction or ammonium sulfate purified PEDNF protein fraction is dialyzed against a buffer such as about 10 mM phosphate buffer, pH 7.2, or other suitable buffer, to remove media and salts from the impure protein sample. PEDNF may be purified by reverse-phase HPLC using a column chromatography medium such as a chromatography medium supplied under the trade name "Vydac C8" by The Separation Group of Hesperia, CA, packed into a column such as a 4.6 x 250 mm column or other suitable reverse-phase HPLC chromatography medium.

The chromatography medium is equilibrated with a solution such as about 0.1 %, by volume, triflouroacetic acid (TFA). The dialyzed impure PEDNF protein fraction or ammonium sulfate purified PEDNF protein fraction is loaded onto the chromatography medium, and the chromatography medium with PEDNF bound to it is washed with an eluant such as 0.1%, by volume, TFA, until all unbound proteins are washed from the chromatography medium. PEDNF is eluted from the chromatograph medium with a linear gradient from about 0.1 % TFA in water to about 95% acetonitrile (CH₃CN), 0.1 % TFA, 5% H₂O. PEDNF elutes as a single peak with a CH₃CN concentration of about 70%.

The eluted PEDNF is concentrated by lyophilization and resolubilized in water or a buffer such as about 10 mM phosphate buffer, pH 7.2, or other suitable buffer, to form a reverse-phase HPLC purified PEDNF protein fraction.

### v. Size-Exclusion HPLC

An impure PEDNF protein fraction, an ammonium sulfate purified PEDNF protein fraction, a cation-exchange HPLC purified PEDNF protein fraction, or a reverse-phase HPLC purified PEDNF protein fraction may be purified by size-exclusion chromatograph using a chromatography medium, such as that supplied under the trade name "Bio-Rad TSK-250" by Bio-Rad of Richmond, CA, packed into a column such as a 7.5 X 300 mm column. The impure PEDNF protein fraction, the ammonium sulfate purified PEDNF protein fraction, the cation-exchange HPLC purified PEDNF protein fraction, or the reverse-phase HPLC purified PEDNF protein fraction is loaded onto the size-exclusion chromatography medium, which has been equilibrated with a buffer such as 0.02 M Tris-HCl, pH 7.0, 0.6 M NaCl. PEDNF-containing fractions are collected and dialyzed against a buffer such as about 10 mM phosphate buffer, pH 7.2 to provide a size-exclusion purified PEDNF protein fraction.

### vi. Heparin Chromatography

An impure PEDNF protein fraction or an ammonium sulfate purified PEDNF protein fraction may also be purified by heparin chromatography. A heparin chromatography medium such as heparin agarose, supplied by Sigma Chemical Co. of St Louis, MO (Cat. No. H-5380), is equilibrated with a buffer such as about 10 mM Tris-HCI, pH 7.5.

An impure PEDNF protein fraction or an ammonium sulfate purified PEDNF protein fraction is dialyzed against a buffer such as about 10 mM Tris, pH 7.5, to remove any salts or media from the samples, and the dialyzed PEDNF solution is applied to the equilibrated heparin agarose. After the PEDNF solution has been applied to the heparin agarose, the heparin agarose is washed with a buffer such as about 10 mM Tris-HCI, pH 7.5, until all unbound proteins are eluted from the heparin agarose. PEDNF is then eluted from the heparin agarose with a buffer such as about 10 mM Tris-HCI, pH 7.5, 0.5 M NaCI.

The eluate containing PEDNF is then diafiltered in an Amicon Diaflo ultrafiltration unit, or is dialyzed against a buffer such as about 10 mM Tris-HCI, pH 7.5, to remove NaCl present in the eluate, thereby providing a heparin purified PEDNF protein fraction.

The above-described purification procedures may use an impure PEDNF protein fraction or an ammonium sulfate purified PEDNF protein fraction as the starting material for the subsequent column purification. Alternatively, a protein fraction which has already been purified by one or more of the described chromatography steps could also be used. Therefore, the purification procedures may be used alone or in combination with each other or with other purification techniques known in the art to produce a PEDNF protein fraction of the desired purity.

### B. Large-Scale Preparation of PEDNF

### i. Ammonium Sulfate Precipitation

Large-scale purification of PEDNF may be by ammonium sulfate precipitation to provide an ammonium sulfate purified PEDNF protein fraction.

An impure PEDNF protein fraction is brought to about 50% saturation with ammonium sulfate by the addition of about 313 g of solid ammonium sulfate per liter of impure PEDNF protein fraction. The ammonium sulfate is preferably added slowly to the impure protein fraction while the solution is stirred, such as with a stir bar on a mechanical stirrer. The ammonium sulfate is added slowly to prevent localized high concentrations of ammonium sulfate which may result in rapid precipitation, and denaturation of proteins present in the impure protein fraction. After all the ammonium sulfate is added, the 50% ammonium sulfate solution is stirred for about 30 min. at 20°C. The 50% ammonium sulfate solution is then centrifuged at about 10,000 g, at 20°C for about 20 min. The supernatant is collected for further processing. Alternatively, the 50% ammonium sulfate solution may be filtered through filter paper, such as Whatman #1, to remove the precipitate. The filtrate is collected for further processing.

The 50% ammonium sulfate solution is then brought to about 70% saturation by the addition of about 137 g/l of ammonium sulfate. The ammonium sulfate is added slowly, and after all the ammonium sulfate is added, the 70% ammonium sulfate solution is stirred for about 30 min. at 20°C. The 70% ammonium sulfate solution is centrifuged or filtered, as described above, and the precipitate is collected.

The ammonium sulfate precipitate is then redissolved in a buffer such as about 10 mM phosphate, pH 7, to form a 55-70% ammonium sulphate fraction, then diafiltered using an Amicon Diaflo ultrafiltration unit, or dialyzed against about 10 mM sodium phosphate, pH 7, to remove the ammonium sulfate from the redissolved 55-70% fraction to provide an ammonium sulfate purified PEDNF protein fraction.

### ii. Anion-Exchange Chromatography

The pI of the PEDNF protein is about 3.9 to about 7.2. Therefore, at a pH of about 7.5, the protein has a net negative charge and binds to an anion-exchange chromatography medium such as DEAE (diethylaminoethyl) cellulose.

For use, an anion-exchange chromatography medium such as DEAE cellulose is equilibrated with a buffer such as about 10 mM Tris, pH 7.5. The PEDNF is applied to the anion-exchange chromatography medium, and the medium is washed with a buffer such as about 10 mM Tris, pH 7.5, until all unbound proteins are eluted from the column. After all the unbound proteins are eluted, PEDNF is eluted with a linear salt gradient from about 0 to about 1 M NaCI in a buffer such as about 10 mM Tris-HCI, pH 7.5. The fractions containing PEDNF are collected and pooled. These fractions are then diafiltered or dialyzed against a buffer such as about 10 mM Tris-HCI, pH 7.5, to remove NaCI, thereby providing an anion-exchange chromatography purified PEDNF protein fraction.

Anion-exchange chromatography may be conducted by either batch or column chromatography techniques.

### iii. Heparin Chromatography

Heparin chromatography may also be used for the large-scale purification of PEDNF. A heparin chromatography medium such as heparin agarose, supplied by Sigma Chemical Co. (Cat. No. H-5380), is equilibrated with a buffer such as about 10 mM Tris-HCI, pH 7.5.

An impure PEDNF protein fraction or an ammonium sulfate purified PEDNF protein fraction is dialyzed against a buffer such as about 10 mM Tris-HCI, pH 7.5, and then applied to the heparin agarose. After the PEDNF solution has been applied to the heparin agarose, the heparin agarose is washed with a buffer such as about 10 mM Tris-HCI, pH 7.5, until all unbound proteins are eluted from the heparin agarose. PEDNF is then eluted from the heparin agarose with a buffer such as about 10 mM Tris-HCI, pH 7.5, 0.5 M NaCI.

The eluant containing PEDNF is collected and diafiltered in an Amicon Diaflo ultrafiltration unit, or dialyzed against a buffer such as about 10 mM Tris-HCI, pH 7.5, to remove the NaCI present in the eluate, to provide a heparin purified PEDNF protein fraction.

The above-described purification procedures may use an impure PEDNF protein fraction or an ammonium sulfate purified PEDNF protein fraction as the starting material for the subsequent column purification. Alternatively, a protein fraction which has already been purified by one or more of the described chromatography steps could also be used. Therefore, the purification procedures may he used alone or in combination with each other or with other purification techniques known in the art to produce a PEDNF protein fraction of the desired purity.

### 3. PEDNF Assays

### A. SDS Gel Electrophoresis

PEDNF may be identified by SDS-polyacrylamide gel electrophoresis on, for example, 7.5%, 10%, 12.5% SDS-polyacrylamide gels. The preparation of such gels has been described by Weber and Osborn, as modified by Laemmli, and the preparation and use of such gels are well known in the art.

The PEDNF protein samples are mixed with about 5 µl of 62.5 mM Tris, pH 6.8, 12% SDS, 0.001 % bromophenol blue, 10% glycerol, and 0.1 M 2-mercaptoethanol, wherein the bromophenol blue is a marker. Molecular-weight marker samples which include molecular-weight standards such as phosphorylase B, bovine serum albumin, ovalbumin, carbonic anhydrase, soybean trypsin inhibitor and lysozyme (such as supplied by Bio-Rad, catalog #161-0304) are included as controls. The PEDNF protein samples and the molecular-weight standards are boiled for about 5 min. and loaded onto SDS-polyacrylamide slab gels. The samples are then subjected to electrophoresis until the bromphenol blue has migrated to the bottom of the gel. At the completion of electrophoresis, the gel is silver-stained, stained with Coomassie blue, or stained by other suitable protein staining methods. The molecular weight of proteins in the PEDNF sample is then compared to the molecular-weight standards.

PEDNF migrates as a protein doublet, with an apparent molecular weight of from about 50,000 to about 55,000 on SDS polyacrylamide gels.

### B. Neuronal Inductivity

PEDNF activity in protein samples may be assayed by its neuronal inductivity. Various concentrations of PEDNF are added to cultures of Y79 retinoblastoma (RB) cells, supplied by the American Type Culture Collection, Access No. HTB 18, of Rockville, MD.

The Y79 RB cells are grown in suspension culture. The cells are harvested by centrifugation for about 5 min. at about 900 rpm at room temperature and resuspended in a serum-free medium such as Dulbecco's modified Eagle's medium supplemented with 5 ug/ml insulin, 5 ug/ml transferrin, 5 ng/ml selenous acid, and 876.6 ug/ml L-glutamine (serum-free medium), which has previously been warmed to about 37°C. The collected cells are resuspended at a concentration of about 10⁶ cells/ml in serum-free medium. About 50 to about 500 ng/ml of PEDNF is added to about 25 ml aliquots of the cells. The cells are incubated for about 7 days at about 37°C, then attached to poly-D-lysine-coated flasks or glass coverslips. The poly-D-lysine-coated flasks are prepared by coating with a solution containing about 200 ug/ml poly-D-lysine (such as that supplied by Sigma, Catalog #P7405) for about 1-24 hours, followed by rinsing with water and serum-free medium.

### i. Cell Analyses

Morphology of attached cells is monitored daily by phase contrast microscopy of living cells using a microscope such as an inverted Diaphot TMD microscope, supplied by Nikon of Tokyo, Japan, or by differential interference contrast microscopy of cells, fixed with a fixative such as about 4% paraformaldehyde in 0.1 M sodium cacodylate buffer, using a microscope, such as a BHS-BH2 microscope, supplied by Olympus of Tokyo, Japan.

Differentiation is assessed by calculating the percentage of cellular aggregates (more than 90 % of Y79 cells plated from suspension culture attach to poly-D-lysine-coated flasks as aggregates containing more than 5 cells) in which cells extend processes at day 1, 3, 7 and 11 after attachment. Experiments are performed in replicates of 3 and are repeated twice.

Expression of neuron-specific enolase (NSE) and neurofilament 200,000 molecular weight protein subunit (NF 200) is monitored by immunofluorescence and viewed by either epifluorescence microscopy (Olympus BHS-BH2 microscope) or microspectrofluorometry (MSA, Farrand Microscope Spectrum Analyzer). Quantification is by 1) visual scoring of intensity of fluorescence at 485 nm excitation as + = weak; + + = moderate; + + + = strong; + + + + = very intense, and 2) microspectrofluorometric analysis (MSA) readings (uA x 100, time constant of about 0.3 seconds; specimen size of approximately 2 mm or about 100 cells/aggregate; target size of about 15 µm or approximately that of 1 cell).

The presence of PEDNF in the protein sample results in the Y79 RB cells, extending neurite-like processes. At a concentration of about 500 ng/ml, about 70% of the cells extend neurite-like processes.

### ii. Differentiation

Cells were cultured as described above. The cells were then monitored daily by phase-contrast microscopy of living cells (Olympus IMT-2) and by differential interference contrast microscopy (Olympus BHS) of cells fixed with a fixative such as about 4% paraformaldehyde. The percentage of differentiating cells is estimated by calculating the number of cellular aggregates containing five or more cells exhibiting neurite outgrowths following 8-10 days of culture on a poly-D-lysine substratum.

With about 50 to about 500 ng/ml PEDNF, approximately 80% of the cells undergo morphological differentiation within about 3 days.

### 4. Characterization of the PEDNF protein

### A. Isolation of PEDNF Peptides

Purified PEDNF, about 500 µg, is concentrated using Centricon 10 microconcentrators (Amicon, Danvers, MA), and then diluted in a suitable digestion buffer such as about 25 mM Tris, pH 8.5, 1 mM EDTA. To the protein sample is added a proteolytic enzyme, such as endoproteinase Lys-C, supplied by Boehringer-Mannheim of Indianapolis, IN. The PEDNF/proteinase mixture is incubated for about 18 hrs. at about 30°C, or until the reaction has gone to completion, i.e., until all the PEDNF is completely digested by the proteinase. Alternatively, the protein may be digested with trypsin in a buffer comprising about 10 mM PBS or by using other proteinases known in the art.

The resulting PEDNF polypeptide fragments are separated by using a separation system such as HPLC on a Vydac C8 reverse-phase column. A 4.6 X 250 mm column is suitable for use in the present invention. The column is equilibrated with about 0.1 % TFA in water. The polypeptides are eluted with about 90% CH₃CN, 0.1% TFA and 5% H₂O.

Polypeptides eluted from the column which are well separated from other polypeptides are collected and subjected to protein sequencing analysis.

### B. Protein Sequencing Analysis

The purified polypeptide fragments are subjected to amino-acid sequence analysis by methods known in the art. Alternatively, the amino-acid sequence analysis is conveniently performed under contract at an amino-acid sequencing facility, such as the Microsequencing Facility of Beckman Research Institute at the City of Hope in Duarte, CA.

### 5. Characterization of the PEDNF Gene

### A. Cloning of the PEDNF Gene

Oligonucleotides are constructed from the sequence derived from the isolated polypeptide of PEDNF on an ABI 392 DNA/RNA Synthesizer or by methods well known in the art.

The oligonucleotides are used as primers for a polymerase chain reaction (PCR) by using a Techne thermal cycler and standard reagents and methodologies, supplied under the trade name "GeneAMP" by Perkin Elmer Cetus of Emeryville CA.

A human fetal eye Charon BS cDNA library is screened by PCR techniques using a Techne thermal cycler and standard reagents and methodologies. The cDNA fragment generated by the reaction is isolated on a 3% NuSieve3:1 gel (FMC Biochemicals) using NA-45 DEAE-cellulose paper (Schleicher and Schull) as described by Sambrook et al. 1989 In: "Molecular Cloning: A Laboratory Manual". 2nd ed. Cold Spring Harbor Press, Cold Spring Harbor, NY, which is incorporated herein by this reference. Briefly, the screening procedure is performed by taking about 1, 5 and 50 µl aliquots of the library and placing them in 600-µl siliconized reaction tubes and then bringing them to a final volume of about 74 µl with double-distilled sterile water. The phage particles are disrupted by incubation at about 70°C for about 5 minutes and then cooling on wet ice.

PCR master mix is made up in a 600-µl reaction tube for 3 reaction tubes as follows:
30 µl of 10X Taq polymerase buffer;
24 µl of dNTP mix; and an appropriate volume of double-distilled water is added to bring the master mix to a final volume of about 78 µl.

The final solution, therefore, comprises about 192 mM KCI, 38.5 mM Tris-HCl, pH 8.3, 51.8 mM MgCl₂, 0.038% (w/v) gelatin, 0.77 mM of each dNTP, and 3.8 µl of each oligonucleotide primer. About 26 µl of master mix is added to each reaction tube. The library aliquots and the master mix solutions are overlayed with about 100 µl of mineral oil and heated to about 94°C for about 5 min. The solutions are then equilibrated to the desired primer annealing temperature.

After the solutions have been equilibrated to the desired primer annealing temperature, about 1.5 µl of Taq polymerase is added to the solution and incubated for about 20 min.

Thermal cycling is continued by incubating: at about 72 °C for about 3 min. for primer extension, however, this primer extension time may be varied if desired; at about 94°C for about 1 min. about 20 sec. to denature the extension product from the template; and at about 37°C for about 2 min. to anneal the primers to a template; and at about 72°C for about 3 min. for primer extension. The "cycle" is the repeated for a total of about 25 to about 30 cycles. At the end of the last cycle, a final primer extension step, of about 7 min. at 72°C is added.

The fragment is labelled with ³²P by random priming using a kit supplied under the trade name "Prime-It Random Primer Labeling Kit" by Stratagene. The labelled probe is used to screen about 200,000 plaque-forming units of the Charon BS cDNA library by methods well known in the art.

Positive clones are isolated, and the DNA purified, with reagents supplied under the trade name "Qiagen Maxi" by Qiagen, Inc. of Studio City, CA.

The inserts within the phage vector are excised with an appropriate restriction enzyme, circularized with T4 DNA ligase supplied by New England Biolabs of Beverly, MA and transformed into competent E. coli Sure cells supplied by Stratagene, Inc. of La Jolla, CA. The cells are then plated on Ampicillin/X-g plates.

White colonies are selected and mini-prepped using a Qiagen plasmid miniprep reagent supplied by Qiagen. Purified plasmids are digested to excise the insert, and the fragments are separated by gel electrophoresis to determine the size of the inserts. A clone with an appropriately-sized insert is then chosen for further investigation.

### B. DNA Sequence Analysis

Sequence analysis is performed with an automated sequencer or by other techniques well known in the art.

### 6. Expression of the PEDNF Gene in Recombinant Cells

Commercial or large-scale production of PEDNF may be achieved by expression of the gene, after cloning into an appropriate vector, in a suitable host cell.
One such suitable vector/host system is the baculovirus/insect cell systems.

In one embodiment of the present invention, the PEDNF gene is cloned into a baculovirus transfer vector such as pAc373, described by Lithgow et al., DNA and Cell Biology, 10, 443-449 (1991); pVL941, described by Ghiasi et al., Virology, 185, 187-194 (1991); or other such baculovirus transfer vectors that are well known by those skilled in the art.

Generally, such vectors comprise the polyhedrin promoter of the Autographa californica nuclear polyhedrosis virus (AcNPV), inserted into a transfer vector such as pAc373, described by Summers and Smith (A manual for baclovirus vector and insect cell culture procedures, Texas Agric. Exp. Station Bull. No. 1555, which is incorporated herein by this reference). Recombinant plasmids are prepared by methods well known in the art, such as those described by Maniatis et al., "Molecular Cloning," Cold Spring Harbor Laboratory, Cold Spring Harbor, NY (1982), which is incorporated herein by this reference.

The host cells, such as S. frugiperda (Sf9), are grown in TC100 medium supplied by GIBCO, supplemented with 10% fetal calf serum. The Sf9 cells are co-transfected with purified infectious AcNPV DNA, about 1 µg, and about 50 µg of the recombinant DNA. Culture supernatants are harvested about 4 days after transfection. Recombinant viruses are identified by plaque hybridization or radiolabelling of the proteins produced.

The above description provides an example of the expression of the PEDNF gene in a vector/host expression system. Other expression systems are known in the art; for example, Saccharomyces cerevisiae has been used in conjunction with a number of vectors such as those described by Silar et al., Gene, 104, 99-102 (1991), Dietrich et al., Eur. J. Biochem., 201, 399-407 (1991), or Akiyoshi-Shibata et al., DNA and Cell Biology, 10, 613-612 (1991); recombinant vaccinia virus vectors in HeLa host cells such as those described by Nakano et al. Gene, 105, 173-178 (1991). Any such methods or other methods known in the art are suitable for use in the present invention for expression of the PEDNF gene.

The PEDNF gene may be expressed as a transported protein where the PEDNF is isolated from the medium in which the recombinant expression host is grown, or may be expressed as an intracellular protein by deleting the leader peptide, in which case the PEDNF is isolated from the host cells. The PEDNF so isolated is then purified by the methods described above.

### 7. Treatment with PEDNF

### A. Treatment of Tumors

PEDNF is administered, alone or in conjunction with other clinical (such as surgical) procedures. PEDNF is administered by intravitreal, subretinal, intravenous ot intramuscular injection or injected or applied at sites of tumor growth. The PEDNF may be administered alone or in conjunction with compounds, such as polylactic acid, which facilitate a slow, "time release" of the PEDNF. Typically, about 0.01 to about 10 µg of PEDNF at a concentration of about 1 to about 100 µg/ml in a physiologic saline solution or other buffered solution is administered per dose, and about 0 to about 10 doses are administered per day. When time-release compounds are included in the composition, they are used at a concentration of about 1 to about 100 µg/ml. Treatment is continued until cessation of tumor growth and/or tumor regression is observed.

Treatment with PEDNF is effective for retinal tumors such as retinoblastoma, other neuronal tumors such as neuroblastoma, or tumors of non-neuronal origin. The treatment results in a cessation or reduction in the rate of cell division and a concomitant reduction in the rate of tumor growth, which in turn results in tumor regression.

### B. Neurotrophic Treatment of Ocular Disease

PEDNF is administered, alone or in conjunction with other clinical (such as surgical) procedures. PEDNF is administered by intravitreal or subretinal injection. The PEDNF may be administered alone or in conjunction with compounds such as polylactic acid which facilitate a slow, "time release" of the PEDNF. Typically, about 0.01 to about 10 µg of PEDNF at a concentration of about 1 to about 100 µg/ml in a physiologic saline solution or other buffered solution is administered per dose, and about 0 to about 10 doses are administered per day. When time-release compounds are included in the composition, they are used at a concentration of about 1 to about 100 µg/ml. Treatment is continued until the progress of the pathology is halted and/or reversed.

Treatment with PEDNF is directed at diseases of the neural retina, retinal pigmented epithelium, and other ocular tissue. Treatment results in enhanced survival and well-being of the photoreceptors and other ocular cells, prolonging their functional life span and delaying the onset of impaired vision and ultimate blindness.

### C. Neurotrophic Treatment of Injured Nerves

PEDNF is administered, alone or in conjunction with other clinical (such as surgical) procedures. PEDNF is administered by intravenous or intramuscular injection or application or injection at the site of nerve injury. The PEDNF may be administered alone or in conjunction with compounds such as polylactic acid which facilitate a slow, "time release" of the PEDNF. Typically, about 0.01 to about 10 µg of PEDNF at a concentration of about 1 to about 100 µg/ml in a physiologic saline solution or other buffered solution is administered per dose, and about 0 to about 10 doses are administered per day. When time-release compounds are included in the composition, they are used at a concentration of about 1 to about 100 µg/ml. Treatment is continued until nerve regeneration is completed.

Treatment with PEDNF is directed at injuries to nerves. Treatment results in promotion of neurite outgrowth and nerve regeneration.

### D. Treatment of Conditions Related to Serine Proteinases

PEDNF is a serine proteinase inhibitor. As such, it is effective in the treatment of conditions caused by serine proteinases or where a serine proteinase inhibitor would be advantageous. Serine proteinases include, but are not limited to, chymotrypsin, trypsin, subtilisin, elastin, thrombin, and plasmin. Therefore, PEDNF is useful as: an anti-coagulant, an anti-thrombotic, an anti-microbial, an anti-fungal, an anti-parasitic, and a contraceptive; in cosmetic preparations as a proteinase inhibitor; as a weight-gain promoter; in treatments for elastosis, vascular disorders involving fibrinoid formation, coagulation disorders, arteriosclerosis, ischemia, arthroses diabetes, emphysema, arthritis, septic shock, lung diseases, excessive complement activation, ulcers. ulcerative colitis, pancreatitis, psoriasis, fibrinolytic disease, arthropathy, hone resorption, hypertension, congestive heart failure, cirrhosis, or allergy caused by proteases.

For use as an anti-coagulant, an anti-thrombotic, an anti-microbial, an anti-parasitic, or a contraceptive, or in treatment of elastosis, vascular disorders involving fibrinoid formation, coagulation disorders, arteriosclerosis, ischemia, arthroses diabetes, emphysema, arthritis, septic shock, lung diseases, excessive complement activation, pancreatitis, psoriasis, fibrinolytic disease, arthropathy, bone resorption, hypertension, congestive heart failure, cirrhosis, or allergy caused by proteases, PEDNF is administered by intravenous or intramuscular injection or site-directed injection or application. The PEDNF may be administered alone or in conjunction with compounds such as polylactic acid which facilitate a slow "time release" of the PEDNF. Typically, about 0.01 to about 10 µg of PEDNF at a concentration of about 1 to about 100 µg/ml in a physiologic saline solution or other buffered solution are administered per dose, and about 0 to about 10 doses are administered per day. When time-release compounds are included in the composition, they are used at a concentration of about 1 to about 100 µg/ml. Treatment is continued until progress of the pathology is halted and/or reversed.

Treatment with PEDNF is directed at injuries to nerves. Treatment results in promotion of neurite outgrowth and nerve regeneration.

For use in cosmetic preparations as a proteinase inhibitor, arthritis or elastosis PEDNF is added to the preparations to a concentration of about 0.01 to about 100 µg/ml.

For use in treatment as a weight-gain promoter, ulcers, ulcerative colitis, or pancreatitis, PEDNF may be administered orally at a concentration of about 0.01 to about 10 µg per kg of body weight per day.

For use as a treatment for conditions such as psoriasis, PEDNF may be administered topically at a concentration of about 0.01 to about 100 µm/ml, formulated in a suitable carrier.

### Example 1

### Establishment of RPE Cell Cultures

RPE cells were harvested from post-mortem human eyes, as described by Pfeffer (1991). The cells were grown in 75 cm flasks with Eagles's minimal essential medium supplemented with 15% fetal calf serum and 5% carbon dioxide at 37°C. Cells were grown to confluence, harvested by trypsinizing the cell monolayer, and resuspended in MEM supplemented with 15% fetal calf serum. A portion of the cells (about 5%) were reseeded into new 75 cm, where the cells were again grown in 5% CO₂ at 37°C.

### Example 2

### Preparation of RPE-conditioned Medium

Confluent cultures of RPE cells were washed extensively with HBSS, before conditioning, to remove serum proteins. Twenty-five ml of serum-free MEM was added, and the cultures were incubated with 5% CO₂ at 37°C for about 48 hours. The conditioned medium was then collected and centrifuged at 1,000 rpm at room temperature, to remove any free cells and other particulate matter, to provide an impure PEDNF protein fraction.

### Example 3

### Purification of PEDNF by SDS Gel Electrophoresis

Proteins contained in human fetal RPE-conditioned medium, prepared as described in Example 2, and in control non-conditioned medium were analyzed on an SDS-polyacrylamide slab gel.

The conditioned and non-conditioned media samples were mixed with an electrophoresis sample buffer (62.5 mM Tris, pH 6.8, 2% SDS, 10% glycerol, 0.001% bromo- phenol blue, and 0.1 M 2-mercaptoethanol). Molecular-weight markers, such as phosphorylase B, bovine serum albumin, ovalbumin, carbonic anhydrase, soybean trypsin inhibitor and lysozyme (such as that supplied by Bio-Rad), were also mixed with an electrophoresis sample buffer. All the samples were denatured at 100°C, in a boiling water bath, for 5 min. and loaded onto a SDS containing 7.5% polyacrylamide gel. The electrophoresis was conducted at 20 mA until the bromphenol blue marker dye migrated to the bottom of the gel.

At the completion of electrophoresis, strips from each outside edge of the gels, which included the markers and a lane each of conditioned and non-conditioned media, were stained with Coomassie blue. The stained protein bands which developed on the stained strips were used to align with the unstained portion of the gel, to locate the proteins present in conditioned medium but absent from non-conditioned medium.

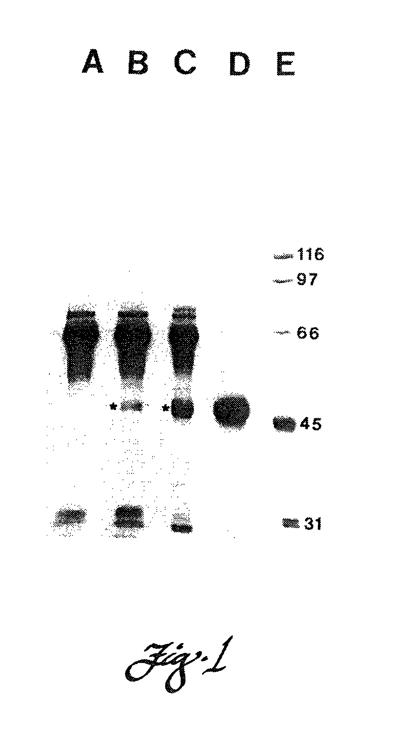

The about 50,000 to about 55,000 molecular-weight PEDNF protein doublet, unique to RPE-CM, were excised, and the proteins were electroeluted from the unstained portion of the gel. A strip of the same gel excised from the region containing bovine serum albumin was treated similarly to serve as a control. The eluant was centrifuged to remove gel fragments, and the supernatant was dialyzed and analyzed by gel electrophoresis to assess its purity. The electrophoretic pattern of the SDS-polyacrylamide gel electrophoresis is shown in FIG. 1.

The electrophoretic patterns of human fetal RPE-CM and non-conditioned control medium show the presence of the prominent about 50,000 to about 55,000 molecular-weight PEDNF doublet unique to the conditioned medium.

### Example 4

### Small Scale Ammonium Sulfate Precipitation

An impure PEDNF protein fraction, prepared as described in Example 2, was divided into six 10 ml aliquots. One of the aliquots was brought to 40% saturation, at 20°C, by the addition of 2.42 g of ammonium sulfate; another aliquot was brought to 50% saturation, at 20°C, by the addition of 3.14 g of ammonium sulfate; another aliquot was brought to 60% saturation, at 20°C, by the addition of 3.90 g of ammonium sulfate; another aliquot was brought to 70% saturation, at 20°C, by the addition of 4.72 g of ammonium sulfate; another aliquot was brought to 80% saturation, at 20°C, by the addition of 5.61 g of ammonium sulfate; and the final aliquot was brought to 90% saturation, at 20°C, by the addition of 6.57 g of ammonium sulfate. The aliquots were mixed until the ammonium sulfate was completely dissolved. The precipitates which formed in each of the tubes were collected by centrifugation at 10,000 rpm for 20 min. The precipitates were each resuspended in 10 mM sodium phosphate buffer, pH 7.5, and dialyzed against 2 l of 10 mM sodium phosphate buffer, pH 7.5, for 24 hours.

The samples were then collected and subjected to SDS-polyacrylamide gel electrophoresis on a 10% SDS-polyacrylamide gel, as described in Example 3.

The results are summarized in Table I.

**Table I**

| % Saturation Ammonium Sulfate | Relative concentration of PEDNF |
|---|---|
| 40 | - |
| 50 | - |
| 60 | + |
| 70 | +++ |
| 80 | - |
| 90 | - |

The results indicate that the PEDNF precipitates with an ammonium sulfate saturation of about 60% to 70%. The small amount of PEDNF in the 50% to 60% sample indicates that some PEDNF is present, and that a suitable ammonium sulfate "cut" is from 50% to 70% saturation. The samples were subjected to SDS-polyacrylamide gel electrophoresis, as described in Example 3. It was estimated that the purity of the PEDNF in the 60% to 70% ammonium sulfate fraction was about 50%.

### Example 5

### Ammonium Sulfate Precipitation

An impure protein fraction, prepared as described in Example 2, was brought to 50% saturation with ammonium sulfate by the addition of 313 g/l of solid ammonium sulfate. The ammonium sulfate was added slowly to the impure protein fraction while the solution was stirred with a stir bar on a mechanical stirrer. After all the ammonium sulfate was added, the 50% ammonium sulfate solution was stirred for 30 min. at 20°C. The 50% ammonium sulfate solution was then centrifuged at 10,000 g, at 20°C for 20 min. The supernatant was collected.

The 50% ammonium sulfate solution was then brought to 70% saturation by the addition of 137 g/l of ammonium sulfate. The ammonium sulfate was added slowly, and after all the ammonium sulfate was added, the 70% ammonium sulfate solution was stirred for 30 min. at 20°C. The 70% ammonium sulfate solution was centrifuged or filtered, as described above, and the precipitate was collected.

The ammonium sulfate precipitate was then redissolved in 10 mM sodium phosphate, pH 7, to form a 55%-70% ammonium sulphate fraction, then diafiltered using an Amicon Diaflo ultrafiltration unit, or dialyzed against 10 mM sodium phosphate, pH 7, to remove the ammonium sulfate from the redissolved 55-70% fraction.

The samples were subjected to SDS-polyacrylamide gel electrophoresis, as described in Example 3. It was estimated that the purity of the PEDNF in the 55% to 70% ammonium sulfate fraction was about 50%.

### Example 6

### Purification of PEDNF by Cation Exchange HPLC

RPE-CM, prepared as described in Example 2, was dialyzed against 10 mM sodium phosphate buffer, pH 6.5, to remove media and salts from the impure protein sample. The dialyzed PEDNF sample was then loaded onto a Brownlee Aquapore CX-300 HPLC chromatography medium, packed into a 4.6 X 30 mm column. The chromatography medium was previously equilibrated with 10 mM phosphate, pH 6.5. The dialyzed RPE-CM was loaded onto the chromatography medium, and the chromatography medium, with PEDNF, was washed with 10 mM phosphate, pH 6.5, until all unbound proteins were washed from the chromatography medium. PEDNF was eluted from the chromatography medium with a linear salt gradient from 0.0 to about 0.5 M NaCI in 10 mM phosphate, pH 6.5. PEDNF, eluted with a NaCI concentration of about 0.25 M. The eluted PEDNF was concentrated by lyophilization and resolubilized in 10 mM phosphate, pH 6.5. The eluted PEDNF was analyzed by SDS gel electrophoresis, as described in Example 3. The eluted PEDNF was estimated to be approximately 70% pure.

### Example 7

### Purification of PEDNF by Cation-Exchange HPLC

Ammonium sulfate-precipitated RPE-CM, prepared as described in Example 5, was dialyzed against 10 mM sodium phosphate buffer, pH 6.5, to remove ammonium sulfate. An 80 µl sample of the dialyzed ammonium sulfate-purified PEDNF protein fraction was loaded onto a Brownlee Aquapore CX-300 chromatography medium, packed into a 4.6 X 30 mm column. The chromatography medium was previously equilibrated with 10 mM phosphate, pH 7.2. The PEDNF/chromatography medium was washed with 10 mM sodium phosphate buffer, pH 7.2, until all unbound proteins were washed from the chromatography medium. PEDNF was eluted from the chromatography medium with a linear salt gradient from 0.0 to about 0.5 M NaCI in 10 mM sodium phosphate buffer, pH 7.2. PEDNF eluted as a single peak with a NaCI concentration of about 0.25 M.

The eluted PEDNF was dialyzed against 10 mM sodium phosphate buffer, pH 7.2, lyophilized, and resolubilized in water. The sample was then subjected to SDS-polyacrylamide gel electrophoresis, as described in Example 3.

The eluted PEDNF was estimated to be about 70% pure.

### Example 8

### Purification of PEDNF by Reverse-Phase HPLC

Ammonium sulfate-precipitated RPE-CM, prepared as described in Example 5, was dialyzed against 10 mM sodium phosphate buffer, pH 6.5, to remove ammonium sulfate. An 80 µl PEDNF-containing sample was then loaded onto a Vydac C8 chromatography medium, packed into a 4.6 X 250 mm column. The chromatography medium was previously equilibrated with 0.1% TFA in water. The PEDNF/chromatography medium was washed with 0.1 % TFA in water until all unbound proteins were washed from the chromatography medium. PEDNF was eluted from the chromatography medium with a linear gradient from 0.0 to about 95% CH₃CN, 0.1% TFA in water, and 5% H₂O.

The eluted PEDNF was dialyzed against 10 mM sodium phosphate buffer, pH 7.2, lyophilized, and resolubilized in water. The sample was then subjected to SDS-polyacrylamide gel electrophoresis, as described in Example 3.

The PEDNF eluted from the column was estimated to be about 80% pure.

### Example 9

### Purification of PEDNF by Reverse-Phase HPLC

A PEDNF-containing sample, partially purified as described in Example 2, was then loaded onto a Vydac C8 chromatography medium, packed into a 4.6 X 250 mm column. The chromatography medium was previously equilibrated with 0.1% TFA in water. The PEDNF/chromatography medium was washed with 0.1% TFA in water until all unbound proteins were washed from the chromatography medium. PEDNF was eluted from the chromatography medium with a linear gradient from 0.0 to about 95% CH₃CN in 0.1 % TFA and 5% H₂O.

The eluted PEDNF was dialyzed against 10 mM sodium phosphate buffer, pH 7.2, lyophilized, and resolubilized in water. The sample was then subjected to SDS-polyacrylamide gel electrophoresis, as described in Example 3.

The PEDNF eluted from the column was estimated to be about 60% pure.

### Example 10

### Purification of PEDNF by Reverse-Phase HPLC

A PEDNF-containing sample, partially purified as described in Example 6, was then loaded onto a Vydac C8 chromatography medium, packed into a 4.6 X 250 mm column. The chromatography medium was previously equilibrated with 0.1 % TFA in water. The PEDNF /chromatography medium was washed with 0.1% TFA in water until all unbound proteins were washed from the chromatography medium. PEDNF was eluted from the chromatography medium with a linear gradient from 0.0 to about 95% CH₃CN in 0.1 % TFA and 5% H₂O.

The eluted PEDNF was dialyzed against 10 mM sodium phosphate buffer, pH 7.2, lyophilized, and resolubilized in water. The sample was then subjected to SDS-polyacrylamide gel electrophoresis, as described in Example 3.

The PEDNF eluted from the column was estimated to be about 80% pure.

### Example 11

### Purification of PEDNF by Reverse-Phase HPLC

A PEDNF-containing sample, prepared as described in Example 6, was loaded onto Brownlee RP-300 chromatography medium, packed into a 4.6 X 250 mm column. The chromatography medium was previously equilibrated with 0.1 % TFA in water. The PEDNF /chromatography medium was washed with 0.1 % TFA in water until all unbound proteins were washed from the chromatography medium. PEDNF was eluted from the chromatography medium with a linear gradient from 0.0 to about 95% CH₃CN in 0.1 % TFA and 5% H₂O.

The eluted PEDNF was dialyzed against 10 mM sodium phosphate buffer, pH 7.2, lyophilized, and resolubilized in water. The sample was then subjected to SDS-polyacrylamide gel electrophoresis, as described in Example 3.

The PEDNF eluted from the column was estimated to be about 90% pure.

### Example 12

### Purification of PEDNF by Size-Exclusion Chromatography

Partially-purified PEDNF, prepared as described in Example 5 or 6, was purified further by chromatography on Bio-Rad TSK-250 chromatography medium, packed into a 7.5 X 300 mm column. A 40 µl sample of resolubilized PEDNF was loaded onto the size-exclusion chromatography medium, which had been previously equilibrated with 20 mM Tris, pH 7.0, 0.6 M NaCl. PEDNF was eluted with 20 mM Tris, pH 7.0, 0.6 M NaCI.

The eluted PEDNF was dialyzed against 10 mM sodium phosphate buffer, pH 7.2, lyophilized, and resolubilized in water. The sample was then subjected to SDS-polyacrylamide gel electrophoresis, as described in Example 3.

The PEDNF eluted from the column was estimated to be about 75% pure.

### Example 13

### Anion-Exchange Chromatography

DEAE cellulose is equilibrated with 10 mM Tris, pH 7.5. PEDNF, prepared as described in Example 5, is applied to the column, and the column is washed with 10 mM Tris, pH 7.5, until all unbound proteins are eluted from the column. After all the unbound proteins are eluted, the PEDNF is eluted with a linear gradient from 0 to about 1 M NaCI in 10 mM Tris-HCI, pH 7.5. The fractions containing PEDNF are collected and pooled. These fractions are then diafiltered against 10 mM Tris-HCI, pH 7.5 to remove NaCl.

### Example 14

### Anion-Exchange Chromatography

DEAE cellulose is equilibrated with 10 mM Tris, pH 7.5. PEDNF, prepared as described in Example 2, is applied to the column, and the column is washed with 10 mM Tris, pH 7.5, until all unbound proteins are eluted from the column. After all the unbound proteins are eluted, the PEDNF is eluted with a linear gradient from 0 to about 1 M NaCI in 10 mM Tris-HCI, pH 7.5. The fractions containing PEDNF are collected and pooled. These fractions are then diafiltered against 10 mM Tris-HCI, pH 7.5 to remove NaCI.

### Example 15

### Cation-Exchange Chromatography

Bio-Rex 70 resin (Bio-Rad) cellulose is equilibrated with 10 mM PBS, pH 7.2. PEDNF, prepared as described in Example 2, is applied to the column, and the column is washed with 10 mM PBS, pH 7.2, until all unbound proteins are eluted from the column. After all the unbound proteins are eluted, the PEDNF is eluted with a linear gradient from 0 to about 1 M NaCl in 10 mM PBS, pH 7.2. The fractions containing PEDNF are collected and pooled. These fractions are then diafiltered against 10 mM Tris-HCI, pH 7.5 to remove NaCI.

### Example 16

### Cation-Exchange Chromatography

Bio-Rex 70 resin (Bio-Rad) cellulose is equilibrated with 10 mM PBS, pH 7.2. PEDNF, prepared as described in Example 5, is applied to the column, and the column is washed with 10 mM PBS, pH 7.2, until all unbound proteins are eluted from the column. After all the unbound proteins are eluted, the PEDNF is eluted with a linear gradient from 0 to about 1 M NaCl in 10 mM PBS, pH 7.2. The fractions containing PEDNF are collected and pooled. These fractions are then diafiltered against 10 mM Tris-HCI, pH 7.5 to remove NaCl.

### Example 17

### Heparin Chromatography

Two ml of heparin agarose was packed into a 0.7 x 10 cm column, washed with 20 ml of 10 mM Tris-HCl, pH 7.5, 3 M NaCl, then equilibrated with 20 ml of 10 mM Tris-HCl, pH 7.5. Twelve ml of a PEDNF solution, prepared as described in Example 2, from a 17-year-old human donor, was dialyzed against 4 l of 10 mM sodium phosphate buffer, pH 7.5, at 4°C for 19 hours. The volume after dialysis was 19 ml. The dialysate was applied to the heparin agarose at a flow rate of 0.4 ml/min. After the PEDNF solution had been applied to the heparin agarose, the heparin agarose was washed with 20 ml of 10 mM Tris-HCl, pH 7.5, to remove unbound proteins from the heparin agarose. PEDNF was then eluted from the heparin agarose with 12 ml of 10 mM Tris-HCI, pH 7.5, 3 M NaCl.

The eluate containing PEDNF was dialyzed against 10 mM Tris-HCI, pH 7.5, to remove the NaCI present in the eluate. The dialyzed eluate was then lyophilized, redissolved in 10 mM sodium phosphate buffer, pH 7.5, and a sample was subjected to 12.5% SDS-polyacrylamide gel electrophoresis, as described in Example 3.

The PEDNF eluate was estimated to be about 60% pure.

### Example 18

### Heparin Chromatography

Two ml of heparin agarose was packed into a 0.7 x 10 cm column, washed with 20 ml of 10 mM Tris-HCI, pH 7.5, 3 M NaCI, then equilibrated with 20 ml of 10 mM Tris-HCl, pH 7.5. One ml of a PEDNF solution, prepared as described in Example 5, was dialyzed against 4 l of 10 mM sodium phosphate buffer, pH 7.5, at 4°C for 19 hours. The volume of the dialysate was 1.3 ml. The dialysate was applied to the heparin agarose at a flow rate of 0.4 ml/min. After the PEDNF solution had been applied to the heparin agarose, the heparin agarose was washed with 20 ml of 10 mM Tris-HCI, pH 7.5, to remove unbound proteins from the heparin agarose. The heparin agarose was then successively eluted with 12 ml of 10 mM Tris-HCI, pH 7.5, 0.5 M NaCI; 12 ml of 10 mM Tris-HCI, pH 7.5, 1 M NaCI; 12 ml of 10 mM Tris-HCI, pH 7.5, 2 M NaCl; and 12 ml of 10 mM Tris-HCI, pH 7.5, 3 M NaCI.

Each of the eluates was dialyzed against 10 mM Tris-HCI, pH 7.5, to remove the NaCI present in the eluate. The dialyzed eluates were then lyophilized and redissolved in 10 mM sodium phosphate buffer, pH 7.5, and a sample of each dialyzed eluate was subjected to 12.5% SDS-polyacrylamide gel electrophoresis, as described in Example 3.

The PEDNF eluate was estimated to he about 70% pure.

### Example 19

### Neuronal Inductivity and Differentiation Activity of Isolated PEDNF

Electroeluted PEDNF was prepared as described in Example 3. To characterize the neuronal inductive activity of the isolated PEDNF, the optimal concen- tration and pre-seeding stimulatory periods were determined. Either 1, 2, 4, 6, 8, or 10 µg/ml of electroeluted PEDNF in serum-free DME, supplemented with 1 mM sodium pyruvate, 0.625 mM HEPES, 6 mM L-glutamine, 1 % non-essential amino acids, 5 µg/ml insulin, 5 µg/ml transferrin, and 5 ng/ml selenous acid, was tested. As a control, RPE-CM, diluted with an equal volume of non-conditioned medium (50% RPE-CM), was also used.

The Y79 RB cells were grown in suspension culture. The cells were harvested by centrifugation for 5 min. at 900 rpm at room temperature and resuspended in serum-free DME medium, which had previously been warmed to 37°C. The cells were collected by centrifugation and resuspended at a concentration of 10⁶ cells/ml in serum-free DME medium. One, 2, 4, 6, 8, or 10 µg/ml electro-eluted PEDNF or 50% RPE-CM was introduced into separate Y79 RB cell cultures. The cells were incubated for 7 days at 37°C, and were then attached to poly-D-lysine-coated flasks. The cells were observed daily by phase-contrast microscopy.

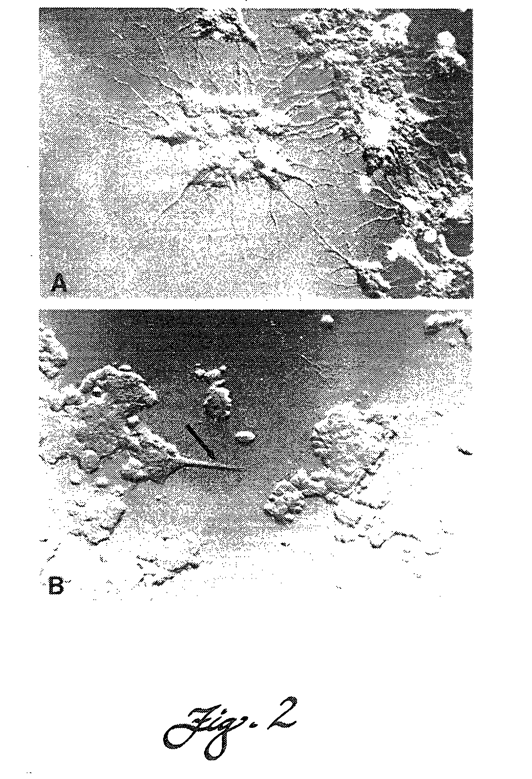

The optimal pre-seeding period required for maximal inductive activity of PEDNF was assessed by treating Y79 RB cell cultures with 2 µg/ml of electroeluted PEDNF for 2, 4, 8, or 16 days prior to seeding onto a poly-D-lysine substratum. In addition, 1 or 2 µg/ml of electroeluted PEDNF was added to attached cultures of cells not previously stimulated in suspension culture. Greater than 80% of Y79 cell aggregates treated with 2 µg/ml electroeluted PEDNF extended arborizing processes within 8-10 days after attachment to a poly-D-lysine substratum; untreated cells showed only minimal signs of neuronal differentiation (FIG. 2).

Y79 cells exhibited maximal differentiative response to electroeluted PEDNF at a protein concentration of 2 µg/ml; the response was reduced at concentrations exceeding 4 µg/ml. The results are shown in Table II.

**Table II**

| PEDNF (µg/ml) | Pre-Attachment Stimulatory Period (days) | Percent Differentiated Cells |
|---|---|---|
| 0 | 7 | 13 ± 8* |
| 1 | 7 | 82 ± 10 |
| 2 | 7 | 80 ± 10 |
| 4 | 7 | 33 ± 10 |
| 6 | 7 | 5 ± 5 |
| 8 | 7 | 0 |
| 10 | 7 | 0 |
| 1 | 0⁺ | 20 ± 14 |
| 2 | 0⁺ | 28 ± 11 |
| 1 µg/ml BSA (control) | 7 | 8 ± 6 |

| | | |
|---|---|---|
| * Standard error | | |
| ⁺ PEDNF added to non-stimulated attached cells, 24 hrs. post-seeding. | | |

A pre-seeding stimulatory period (in suspension culture) of 8 days, in the presence of this factor, results in maximal differentiation of Y79 cells at day 10 post-attachment. Decreased frequencies of differentiation (less than 50%) are noted with both shorter and longer pre-seeding inductive periods. In addition, cells not previously stimulated with PEDNF prior to attachment, exhibit a low frequency of neuronal differentiation (approximately 20%) if 1-2 µg/ml electroeluted protein is added post-attachment. This response, however, is relatively slow, i.e., less than 15 days. Control BSA-treated cultures exhibit less than 10% differentiation, comparable to cells exposed only to non-conditioned media.

PEDNF induced a high degree of neuronal differentiation in human retinoblastoma cells. Long ramifying neuritic processes were induced to extend from aggregates of stimulated cells; concomitant increases in the expression of the neuronal marker molecules, neuron-specific enolase, and the 200,000 molecular weight neurofilament protein were also observed. The expression of the neuronal phenotype in Y79 cells appears to involve three sequential events: 1) stimulation of cells in suspension culture by PEDNF; 2) attachment of stimulated cells to a substratum; followed by 3) neurite outgrowth of attached, stimulated cells. Since only 20% differentiation was observed when non-stimulated, attached cultures were treated with PEDNF, commitment to neuronal differentiation in Y79 cells appears to precede cell attachment and neurite outgrowth and may be initiated during the stimulatory period.

### Example 20

### Comparison of the Effects of RPE-CM from Different Species

Cell cultures were established as described in Example 1, except the eyes from which the cells were derived were either fetal humans, adult humans, adult rats, fetal rats, or embryonic chickens.

RPE-CM was prepared from each of the cell cultures, as described in Example 2. Fetal human cell cultures which were newly established (short-term cultures) and cultures which had been established for 6 months (long-term cultures) were included.

The differentiation activity of the conditioned media was performed as described in Example 19.

The results of experiments monitoring the effects of RPE-CM from various species are summarized in Table III.

**Table III**

| Source of RPE-Conditioned Medium Aggregates | % Differentiated |
|---|---|
| Human (fetal, short-term cultures) | 88 ± 3* |
| Human (fetal, long-term cultures) | 50 ± 8 |
| Human (adult) | 55 ± 9 |
| Rat (adult) | 20 ± 11 |
| Rat (fetal) | 42 ± 7 |
| Chicken (embryonic) | 86 ± 7 |

| | |
|---|---|
| * Standard error | |

The table summarizes the inductive activity of a variety of RPE-conditioned media on Y79 cells. Indicated are the percentages of 10 days attached aggregates (more than 5 cells/aggregate) exhibiting neurite outgrowth after 7 days' stimulation with 50% RPE-CM from the various species. One hundred aggregates were counted from each sample in replicates of three.

The greatest differentiative response in Y79 cells is induced by human fetal RPE-CM (short-term cultures) and embryonic chicken RPE-CM, both of which induce neuronal differentiation in greater than 80% of cellular aggregates when used at a 50% concentration. In contrast, decreased frequencies (less than 50%) of cellular differentiation are noted for conditioned media from long-term (12-18 months) cultures of human fetal RPE and adult human RPE. Only 40% neuron-like differentiation is induced by fetal rat RPE-CM and 20% by adult rat RPE-CM.

Conditioned media from human fetal and embryonic chicken RPE cells contain similar neurotrophic activity, while those from adult cultures (chicken and rat) and long-term cultures of human fetal RPE-CM are less effective in promoting the neuronal phenotype in Y79 cells. It is possible that mature RPE cells no longer secrete this neurotrophic factor, or they secrete reduced quantities which are less effective in the culture conditions utilized. The fact that some trophic activity is seen in RPE-conditioned media from other species suggests that PEDNF, or a similar factor, is also secreted by RPE cells of other species and may be generally important to normal retinal development and function.

### Example 21

### Two-Dimensional Gel Electrophoresis

Two-dimensional gel analysis was conducted by the method described by O'Farrell, J. Biol. Chem., 250, 4007-4021 (1975) and Jones, J., J. Exp. Med, 14b, 1251-1279 (1977). Silver-staining was performed using a Bio-Rad silver-stain plus kit. Twenty µg of PEDNF, purified by cation-exchange and size-exclusion HPLC, was loaded onto the IEF tube gel.

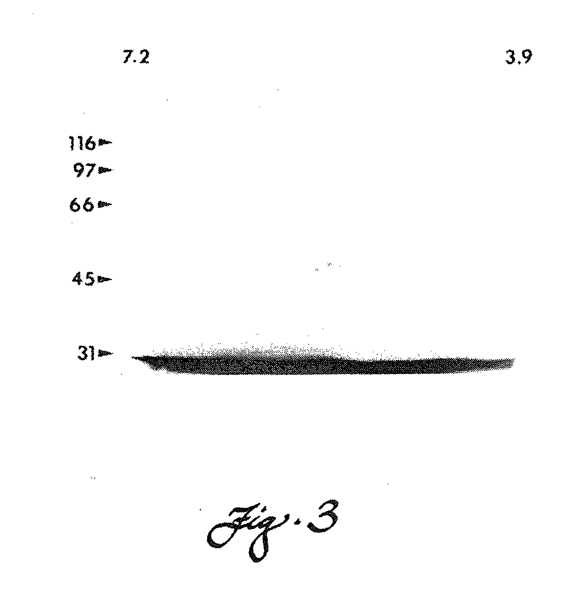

Two-dimensional electrophoretic analysis of HPLC-purified PEDNF reveals the presence of four closely-grouped molecular species (FIG. 3). The four species vary slightly in apparent molecular weight, but all are in the 50,000 molecular-weight region of the gel, consistent with the previously identified molecular weight of PEDNF. The resolved species also vary slightly in isoelectric point, suggesting slight variations in post-translational modifications. Amino-acid sequence analysis (see Example 22), however, indicated the presence of only a single polypeptide.

### Example 22

### Isolation of PEDNF Specific Peptides and Amino-Acid Sequences

180 µg of purified PEDNF (purified as described in Examples 2, 6, and 12) was concentrated using a Centricon 10 microconcentrator, supplied by Amicon of Danvers, MA, and then diluted in 25 mM Tris, pH 8.5, 1 mM EDTA. To the protein sample was added endoproteinase Lys-C. The PEDNF/proteinase mixture was incubated for ahout 18 hrs. at 30°C to digest the PEDNF.

The resulting PEDNF polypeptide fragments were separated using by HPLC on a Vydac C8 reverse-phase HPLC packed into a 4.6 X 250 mm column. The column was equilibrated with 0.1% TFA in water. The polypeptides were eluted with 90% CH₃CN, 0.1 % TFA in water.

Polypeptides eluted from the column which are well separated from other polypeptides were collected and subjected to protein sequencing analysis. The amino-acid sequence analysis was performed under contract at the Microsequencing Facility of Beckman Research Institute at the City of Hope.

The amino-acid sequences for the isolated polypeptides were determined to be:

Sequencing of PEDNF-13 yielded 19 residues of unequivocal sequence. PEDNF-14 yielded 26 residues, 25 of which were unequivocal. The identification of residue 23 in PEDNF-14 as tryptophan was not absolutely certain.

### Example 23

### Isolation of PEDNF Specific Peptides and Amino Acid Sequence

PEDNF, purified as described in Examples 2, 6, and 12, was reduced and alkylated. The sample was dried, redissolved in 50 µl of CRA buffer (8 M urea, 0.4 M ammonium bicarbonate, pH 8.0), and 5 µl of 45 mM DTT (Calbiochem) was added. After heating at 50°C for 15 minutes, the solution was cooled, and 5 µl of 100 mM iodoacetic acid (Sigma Chemical Co.) was added. After 15 minutes, the solution was diluted to a concentration of 2 M urea and subjected to trypsin digestion, supplied by Boheringer-Mannheim, using an enzyme:substrate ratio of 1:25 (wt/wt), for 22 hrs at 37°C.

Tryptic peptides were separated by narrow-bore reverse-phase HPLC on a Hewlett-Packard 1090 HPLC, equipped with a 1040 diode array detector, using a Vydac 2.1 mm x 150 mm C18 column. Buffer A was 0.06% trifluoroacetic acid/H₂O, and buffer B was 0.055% trifluoroacetic acid/acetonitrile, a gradient of 5% B at 0 min., 33% B at 63 min., 60% B at 95 min., and 80% B at 105 min., with a flow rate of 150 µl/min., was used. Chromatographic data at 210, 277 nm and UV spectra from 209 to 321 nm of each peak were obtained. Samples for amino terminal sequence analysis were applied to a polybrene precycled glass fibre filter and subjected to automated Edman degradation at the Harvard Microchemical Facility in Boston, MA, on an ABI model 477A gas-phase protein sequencer using program NORMAL 1. The resulting phenylthiohydantoin amino acid fractions were manually identified using an on-line ABI Model 120A HPLC and Shimadzu CR4A integrator.

The sequences of the isolated peptides were determined to be:

### Example 24

### Comparison of the PEDNF Peptides to Rat Serine Proteinase Inhibitor Sequences

PEDNF-13 shows significant sequence. Homologous molecules include rat serine protease inhibitors 1 and 2, a rat hepatocyte growth hormone-regulated protein, a rat thyroid hormone-regulated protein, and mouse contrapsin. Human, monkey, sheep, and mouse alpha-1 antitrypsin, and porcine alpha-1-antichymotrypsin, show significant but somewhat less homology. PEDNF-14 shows homology with different protease inhibitors, but the degree of homology is less than that observed for PEDNF-13. These include human plasma protease C1 inhibitor and human alpha-2-antiplasmin inhibitor. These results suggest that PEDNF may function as a protease inhibitor, but that it is molecularly distinct from such inhibitors which have been described previously.

PEDNF shows significant homology with serpins, the family of serine protease inhibitors that share a common reactive center near the C-tenninal end, which serves as an exposed binding site that acts as bait for target serine proteinases. It is of interest that a number of known members of the serpin family have been shown to have neurotrophic effects on a variety of neuronal cell types. For example, glial-derived nexin (GDN) promotes neurite outgrowth in neuroblastoma cells, as do a number of other protease inhibitors, such as hirudin and leupeptin. Protease inhibitors also stimulate neuronal differentiation in cells of dorsal root ganglia, sympathetic neurons, and hippocampal pyramidal neurons. It is also of interest that the production of proteinases and protease inhibitors is stimulated by a number of known growth factors. While it remains to be determined if PEDNF has protease inhibitor activity, it is likely, since inhibitory activity has been shown to be necessary for the neurite-promoting activity of glial derived nexin. It has been suggested that the formation of a stable protease-GDN complex, and a consequent conformational change in GDN and/or the associated protease, is necessary for the neurite-promoting activity of GDN. PEDNF may well function similarly, as there are known proteinases present in the interphotoreceptor matrix and in the developing neural retina.

### Example 25

### Cloning of the PEDNF Gene

Oligonucleotides were constructed against PEDNF 13: determined in Example 22, and PEDNF 2: determined in Example 23.

The oligonucleotides were prepared on an ABI 392 DNA/RNA Synthesizer and used as primers in a polymerase chain reaction (PCR). A human fetal eye Charon BS cDNA library, donated by Dr. A. Swaroop, was amplified once by the method described by Sambrook et al. (1989) Molecular Cloning: A Laboratory Manual. 2nd Ed. (Cold Spring Harbor Press, Cold Spring Harbor, NY) and screened by PCR as described by Friedman et al. (1990, in Screening of λgt 11 libraries in PCR protocols: a guide to methods and applications. Innis, Gelfand, Sninsky and White, eds., Academic Press, pp. 253-260) using a Techne thermal cycler and standard reagents (GeneAMP, Perkin Elmer Cetus), except that MgSO₄ was used at 3 mM.

The recovered fragment was isolated on a 3% NuSieve 3:1 gel (FMC Biochemicals) using NA-45 DEAE-cellulose paper (Schleicher and Schull) and labelled with ³²P by random priming (Prime-It Random Primer Labeling Kit, Stratagene).

This probe was used to screen 200,000 pfus of the same library (10). Positive clones were isolated as described by Sambrook et al supra, and the DNA was purified with Qiagen Maxi preparation protocols (Qiagen, Inc.).

The inserts were cut out with Not1 (BRL, Gaithersburg, MD) circularized with T4 DNA ligase (New England Biolabs), transformed into competent E. coli Sure cells (Stratagene, Inc.), and plated out on 12.5 µg/ml Ampicillin/40 µg/ml X-gal agar plates.

White colonies were selected and mini-prepped (Qiagen plasmid mini-prep protocol). Purified plasmids were digested with EcoR1/HindIII (BRL) and run on a 0.7% agarose gel to determine the size of the inserts.

### Example 26

### DNA Sequence Analysis

One of the identified clones which contained an insert corresponding to the PEDNF gene was selected for mapping and subsequent sequencing using a USB Sequenase 2.0 protocol and reagents.

### Example 27

### Treatment of Retinal Tumors

PEDNF is administered, alone or in conjunction with clinical (such as surgical) procedures. PEDNF is administered by intravitreal or subretinal injection. The PEDNF may be administered alone or in conjunction with compounds such as poly(lactic acid) which facilitate a slow, "time release" of the PEDNF. Typically, about 0.01 to about 10 µg of PEDNF at a concentration of about 1 to about 100 µg/ml in a physiologic saline solution or other buffered solution is administered per dose, and about 0 to about 10 doses are administered per day. When time-release compounds are included in the composition, they are used at a concentration of about 1 to about 100 µg/ml. Treatment is continued until tumor progression is halted or reversed.

Treatment with PEDNF is effective for retinal tumors such as retinoblastoma, other neuronal tumors such as neuroblastoma, or tumors of non-neuronal origin. The treatment results in a cessation or reduction in the rate of cell division and a concomitant reduction in the rate of tumor growth, which in turn results in tumor regression.

### Example 28

### Neurotrophic Treatment of Ocular Disease

PEDNF is administered, alone or in conjunction with clinical (such as surgical) procedures. PEDNF is administered by intravitreal or subretinal injection. The PEDNF may be administered alone or in conjunction with compounds such as poly(lactic acid) which facilitate a slow, "time release" of the PEDNF. Typically, about 0.01 to about 10 µg of PEDNF at a concentration of about 1 to about 100 µg/ml in a physiologic saline solution or other buffered solution is administered per dose, and about 0 to about 10 doses are administered per day. When time-release compounds are included in the composition, they are used at a concentration of about 1 to about 100 µg/ml. Treatment is continued until the pathology is halted or reversed.

Treatment with PEDNF is directed at diseases of the neural retina, retinal pigmented epithelium, and other ocular tissue. Treatment results in enhanced survival and well-being of the photoreceptors and other ocular cells, prolonging their functional life span and delaying the onset of impaired vision and ultimate blindness.

### Example 29

### Neurotrophic Treatment of Injured Nerves

PEDNF is administered, alone or in conjunction with clinical (such as surgical) procedures. PEDNF is administered by intravitreal or subretinal injection. The PEDNF may be administered alone or in conjunction with compounds such as poly(lactic acid) which facilitate a slow, "time release" of the PEDNF. Typically, about 0.01 to about 10 µg of PEDNF at a concentration of about 1 to about 100 µg/ml in a physiologic saline solution or other buffered solution is administered per dose, and about 0 to about 10 doses are administered per day. When time-release compounds are included in the composition, they are used at a concentration of about 1 to about 100 µg/ml. Treatment is continued until nerve regeneration is completed.

Treatment with PEDNF is directed at injuries to nerves. Treatment results in promotion of neurite outgrowth and nerve regeneration.

### Example 30

### Transfection of Bacterial Cells

Competent E. coli Sure cells are mixed with the ligation mixture and incubated on ice for 60 min. The mixture is then incubated at 42°C for 2 min., then 800 µl of 2% BACTO Tryptone, 2% BACTO Yeast Extract (both supplied by DIFCO LABORATORIES), 10 mM NaCI, 10 mM MgSO₄, 20 mM glucose are added and the mixture incubated at 37°C for 60 min. 50 to 500 µl of the DNA mixtures are then spread on selection plates containing 12.5 µg/ml Ampicillin.

The recombinant colonies are screened by miniprepping and digestion of the isolated DNA. Positive colonies containing the appropriate inserts are then prepared by growing the appropriate colony in 200 ml of Luria Broth plus 12.5 µg/ml ampicillin with shaking at 37°C overnight. After the overnight incubation, the cultures are transferred to centrifuge bottles and centrifuged at 2500 rpm for 10 min. The supernatant is removed and the cells are resuspended in 30 ml of STET buffer (0.23 M sucrose, 5% Triton-X-100, 20 mM EDTA, 50 mM Tris-HCI, pH 8) at room temperature. Five µl of 10 mg/ml lysozyme is added and the mixture is swirled and incubated for about 5 min. at room temperature. Each flask is then gently swirled directly over a flame until the cells begin to coagulate and turn white. The mixture is then transferred to a boiling-water bath for about 45 sec. The solution is then cooled in an ice-water bath for 2 min., and the mixture is transferred to centrifuge tubes and centrifuged for 15 min. at 16,000 rpm.

The supernatant is then transferred to a clean container. Two volumes of 100% ethanol are added, mixed, and the DNA precipitated at -70°C for 20 min. The precipitate is collected by centrifugation at 2,500 g for 15 min. The ethanol supernatant is removed, and the pellet is washed with about 10 ml of cold (-20°C) 90% ethanol. 2.5 ml of extraction buffer (0.2 M Tris, pH 7.5, 0.08 M EDTA, and 0.2 M KCI) is added to the pellet, and it is resuspended, at 4°C. 100 µl of 10 mg/ml RNAse, dissolved in 0.1X TE (1 mM Tris-HCI, 0.1 mM EDTA, pH 7.6) and pretreated by boiling for 10 min.

### Example 31

### Insect Cell Cultures

Sf9 cells are seeded into spinner flasks, containing Grace's Antheraea medium supplied by GIBCO of Grand Island, NY, to an initial density of 1 x 10⁶ cells/ml and incubated at 27°C with constant stirring at 50 rpm. The cells are subcultured when they reach a density of 2.5 x 10⁶ cells/ml, approximately 2 to 3 times a week, and are diluted about 1 in 5.

### Example 32

### Cloning Genes into pAC373

Two µl of pAC373 are combined with 25 µl of 10X Bam HI restriction enzyme buffer, 20 units of Bam HI and sterile distilled water to bring the volume to 250 µl, and incubated at 37° for at least 3 hours. After the plasmid is digested. It is dephosphorylated by adding one unit of calf intestinal alkaline phosphatase (CAP)/µg of DNA and incubated for 30 min. at 37°C. The CAP is then inactivated by adding EDTA to 25 mM and SDS to 0.5% and incubated at 65°C for 15 min. The solution is then extracted with an equal volume of phenol/chloroform/ isoamyl alcohol (25:24:1).

The aqueous phase is collected, and 125 µl of 7.5 M ammonium acetate and 800 µl of 95% ethanol are added and mixed. The DNA is precipitated at -70°C for 10 min. The precipitate is then collected by centrifugation by centrifugation at 12,000 rpm for 10 min. The pellet is rinsed with (-20°C) 90% ethanol. The ethanol is then removed and the DNA is resuspended in 50 µl of 10 mM Tris-HCI, 1 mM EDTA, pH 7.6 (IX TE).

### Example 33

### Ligation of PEDNF to pAC373

A purified DNA fragment containing the PEDNF gene is ligated to the transfer vector. About 200 ng of digested pAC373 are mixed with an equal molar concentration of PEDNF containing fragment. Ligation buffer (50 mM Tris-HCI, pH 7.4, 10 mM MgCl₂, 10 mM dithiothreitol, 0.5 mM spermidine, 2 mM ATP, 2.5 mM hexamine cobalt chloride and 20 µg/ml BSA) and 10 units of T4 DNA ligase is added. Water is added to a total volume of 10 µl. The mixture is incubated at 14°C for 3 hrs.

### Example 34

### Transferring Genes into the AcMNPV Genome

Sf9 cells are seeded into 25 cm flasks at a density of 2.0 x 10⁶ cells/flask in Grace's Antheraea medium. The cells are allowed to attach for at least one hr. One µg of MNPV DNA is added to 2 µg of plasmid DNA, which contains the PEDNF gene. The medium is removed from the flask and replaced with 0.75 ml of Grace's medium plus 10% fetal bovine serum and antibiotics (50 µg/ml gentamycin sulfate, 2.5 µg/ml amphotericin). 7.5 ml of transfection buffer (25 mM HEPES, pH 7.1, 140 mM NaCI, 125 mM CaCl₂) is added to the DNA solution and mixed. The DNA solution is added to the Grace's medium, already in the cell culture flasks.

Calcium phosphate precipitates form due to the calcium chloride in the transfection buffer and the phosphate in the medium. The flasks are incubated at 27°C for 4 hrs., after which time the medium is removed from the flasks, and the cells are rinsed with fresh TNM-FH (Grace's medium plus 3.3 g/l YEASTOLATE and 3.3 g/l Lactalbumin hydrolysate, both from DIFCO LABORATORIES) plus 10% fetal bovine serum and antibiotics, as described previously, and 5 ml of TNM-FH plus 10% fetal bovine and antibiotics, as described previously, is added to the cells. The cells are incubated for 4-6 days. When the infection is at an advanced stage, the virus is plated on fresh monolayers, and the recombinant viruses are plaque-purified.

### Example 35

### Identification of Recombinant Proteins

Plaque-purified virus is screened by radiolabelling. The recombinant proteins are identified on SDS-PAGE gels. Sf9 cells are seeded at 6 x 10⁵ cells/well in a 24-well culture plate. The cells are allowed to attach for an hour. The medium is then removed and overlaid with medium containing the viral stock and incubated for 1 hr. at 27°C, after which time the viral inoculum is removed and replaced with 500 µl of complete medium. The cells are incubated for 48 hrs. at 27°C. At the end of this incubation, the medium is removed. 200 µl of methionine-free Grace's medium is added to the cells, and the cells are incubated for 60 min., after which time the medium is replaced with 200 µl of fresh methionine-free Grace's medium to which 10 µCi of ³⁵S-methionine is added. Cells are incubated at 27°C for 6 hrs. and then harvested by centrifugation. The supernatant is collected, and the cells are resuspended in 62.5 mM Tris, pH 6.8, 2% SDS, 10% glycerol, 0.001% bromophenol blue, and 0.1 M 2-mercaptoethanol. An equal volume of 126 mM Tris, pH 6.8, 4% SDS, 20% glycerol, 0.002% bromophenol blue, and 0.2 M 2-mercaptoethanol is added to the supernatant. Samples are then boiled for 3 min. and then they are subjected to electrophoresis and autoradiography of the gel to identify proteins secreted into the medium and proteins that are not secreted. Controls of uninfected cells and cells infected with wild-type virus are included.

The above descriptions of exemplary embodiments of retinal pigmented epithelium derived neurotrophic factor are for illustrative purposes. Because of variations which will be apparent to those skilled in the art, the present invention is not intended to be limited to the particular embodiments described above. The scope of the invention is defined by the following claims.

## Claims

1. A method for purifying a PEDNF comprising the properties of: (1) being a glycoprotein with a molecular weight of 50,000 to 55,000; (2) having a pl of 3.9 to 7.2; and (3) inducing neuronal differentiation when applied to embryonic or immortalized cells, further comprising an amino acid composition of: Asp 25, Asn 11, Thr 28, Ser 38, Glu 26, Gin 15, Pro 29, Gly 23, Ala 25, Val 25, Met 7, lie 22, Leu 57, Tyr 10, Phe 18, His 8, Lys 27, Trp 3, and Arg 18, said method comprising the steps of:
(a) providing an impure protein fraction containing PEDNF;
(b) precipitating contaminating proteins from the impure protein fraction containing PEDNF by bringing the impure protein fraction containing PEDNF to 50% to 60% saturation with ammonium sulfate to provide a 50% to 60% ammonium sulfate protein fraction, and/or precipitating PEDNF from the impure protein fraction containing PEDNF by bringing the impure protein fraction containing PEDNF to 70% to 80% saturation with ammonium sulfate to provide a 70% to 80% ammonium sulfate protein fraction;
(c) applying the product of step (b) containing PEDNF to a cation-exchange chromatography medium;
(d) washing the cation-exchange chromatography medium to elute any unbound proteins;
(e) eluting PEDNF from the cation-exchange chromatography medium; and
(f) collecting the PEDNF containing fractions to provide a cation-exchange chromatography-purified PEDNF.

2. The method according to claim 1, wherein in step (b) PEDNF is precipitated from the 50% to 60% ammonium sulfate fraction by bringing the 50% to 60% ammonium sulfate protein fraction to 70% to 80% saturation with ammonium sulfate to provide a 50% to 80% ammonium sulfate protein fraction.

3. The method according to claim 1 or 2 further comprising:
(a) applying the cation-exchange chromatography purified PEDNF protein fraction to a size-exclusion chromatography medium;
(b) eluting the proteins from the size-exclusion chromatography medium; and
(c) collecting the PEDNF-containing fractions.

4. The method according to any one of claims 1 to 3, wherein the impure protein fraction containing PEDNF comprises retinal pigmented epithelium conditioned media.

5. The method according to any one of claims 1 to 3, wherein the impure protein fraction containing PEDNF comprises an extract of a non-human organism transformed with a recombinant DNA molecule comprising a PEDNF gene in a form capable of expression of the PEDNF gene.

6. The method according to any one of claims 1 to 3, wherein the impure protein fraction containing PEDNF comprises a medium in which a non-human organism, transformed with a recombinant DNA molecule comprising a PEDNF gene in a form capable of expression of the PEDNF gene, is grown.

## Patentansprüche

1. Verfahren zur Reinigung von PEDNF, der die folgenden Eigenschaften umfasst: (1) er ist ein Glycoprotein mit einem Molekulargewicht von 50.000 bis 55.000; (2) er besitzt einen pl-Wert von 3,9 bis 7,2; und (3) induziert die neuronale Differenzierung, wenn er für embryonale oder immortalisierte Zellen verwendet wird, wobei der PEDNF weiterhin eine Aminosäurezusammensetzung aus: Asp 25, Asn 11, Thr 28, Ser 38, Glu 26, Gin 15, Pro 29, Gly 23, Ala 25, Val 25, Met 7, lle 22, Leu 57, Tyr 10, Phe 18, His 8, Lys 27, Trp 3 und Arg 18 umfasst, wobei das Verfahren die folgenden Schritte umfasst:
(a) Bereitstellen einer unreinen Proteinfraktion, die PEDNF enthält;
(b) Ausfällen der verunreinigenden Proteine aus der unreinen, PEDNF enthaltenden Proteinfraktion durch Einstellen der unreinen, PEDNF enthaltenden Proteinfraktion auf eine Ammoniumsulfatsättigung von 50% bis 60%, um eine 50 bis 60%-ige Ammoniumsulfat-Proteinfraktion zu erhalten, und/oder Ausfällen von PEDNF aus der unreinen, PEDNF enthaltenden Proteinfraktion durch Einstellen der unreinen, PEDNF enthaltenden Fraktion auf eine Ammoniumsulfatsättigung von 70% bis 80%, um eine 70 bis 80%-ige Ammoniumsulfat-Proteinfraktion zu erhalten;
(c) Aufbringen des PEDNF enthaltenden Produkts aus Schritt (b) auf ein Kationenaustauschchromatographiemedium;
(d) Waschen des Kationenaustauschchromatographiemediums, um jegliche ungebundene Proteine zu eluieren;
(e) Eluieren des PEDNF von dem Kationenaustauschchromatographiemedium;
und
(f) Sammeln der PEDNF enthaltenden Fraktionen, um einen durch Kationenaustauschchromatographie gereinigten PEDNF bereitzustellen.

2. Verfahren nach Anspruch 1, wobei in Schritt (b) PEDNF aus einer 50 bis 60%-igen Ammoniumsulfatfraktion durch Einstellen der 50 bis 60%-igen Ammoniumsulfatproteinfraktion auf eine Ammoniumsulfatsättigung von 70% bis 80% ausgefällt wird, um eine 50 bis 80%-ige Ammoniumsulfatproteinfraktion bereitzustellen.

3. Verfahren nach Anspruch 1 oder 2, ferner umfassend:
(a) Aufbringen der durch Kationenaustauschchromatographie gereinigten PEDNF-Proteinfraktion auf ein Größenausschlusschromatographiemedium;
(b) Eluieren der Proteine aus dem Größenausschlusschromatographiemedium;
und
(c) Sammeln der PEDNF enthaltenden Fraktionen.

4. Verfahren nach einem der Ansprüche 1 bis 3, wobei die unreine Proteinfraktion, die PEDNF enthält, durch pigmentiertes Netzhautepithel konditionierte Medien umfasst.

5. Verfahren nach einem der Ansprüche 1 bis 3, wobei die unreine Proteinfraktion, die PEDNF enthält, einen Extrakt eines nicht-menschlichen Organismus umfasst, der mit einem ein PEDNF-Gen umfassenden rekombinanten DNA-Molekül in einer solchen Form transformiert ist, dass das PEDNF-Gen exprimiert werden kann.

6. Verfahren nach einem der Ansprüche 1 bis 3, wobei die unreine Proteinfraktion, die PEDNF enthält, ein Medium umfasst, in dem ein nicht-menschlicher Organismus gezüchtet wird, der mit einem ein PEDNF-Gen umfassenden rekombinanten DNA-Molekül in einer solchen Form transformiert ist, dass das PEDNF-Gen exprimiert werden kann.

## Revendications

1. Méthode pour purifier un PEDNF comprenant les propriétés de: (1) être une glycoprotéine ayant un poids moléculaire de 50,000 à 55,000 ; (2) avoir un pI de 3,9 à 7.2 ; et (3) induire la différentiation neuronale lorsqu'il est appliqué à des cellules embryonnaires ou immortalisées, comprenant en outre une composition en acides aminés de : Asp25, Asn11, Thr28, Ser38, Glu26, Gln15, Pro29, Gly23, Ala25, Val25, Met7, Ilc22, Leu57, Tyr10, Phe18, His8, Lys27, Trp3 et Arg18, ladite méthode comprenant les étapes de :
(a) fournir une fraction protéique impure contenant PEDNF ;
(b) précipiter les protéines contaminantes de la fraction protéique impure contenant PEDNF en amenant la fraction protéique impure contenant PEDNF à 50 à 60% de saturation avec du sulfate d'ammonium pour fournir une fraction protéique à 50 à 60% de sulfate d'ammonium, et/ou précipiter PEDNF de la fraction protéique impure contenant PEDNF en amenant la fraction protéique impure contenant PEDNF à 70 à 80% de saturation avec du sulfate d'ammonium pour fournir une fraction protéique à 70 à 80% de sulfate d'ammonium ;
(c) appliquer le produit de l'étape (b) contenant PEDNF sur un support chromatographique d'échange de cations ;
(d) laver le support chromatographique d'échange de cations pour éluer toutes protéines non liées ;
(e) éluer PEDNF du support chromatographique d'échange de cations : et
(f) récupérer les fractions contenant PEDNF pour fournir un PEDNF purifié par chromatographie d'échange de cations.

2. Méthode selon la revendication 1, dans laquelle PEDNF est précipité dans l'étape (b) à partir de la fraction à 50 à 60% de saturation en sulfate d'ammonium en portant la fraction protéique à 50 à 60% de saturation en sulfate d'ammonium à 70 à 80% de saturation en sulfate d'ammonium pour fournir une fraction protéique à 50 à 80% de saturation en sulfate d'ammonium.

3. Méthode selon la revendication 1 ou 2, comprenant en outre:
(a) appliquer la fraction protéique PEDNF purifiée par chromatographie d'échange de cations sur un support chromatographique d'exclusion de taille ;
(b) éluer les protéines du support chromatographique d'exclusion de taille ; et
(c) récupérer les fraction contenant PEDNF.

4. Méthode selon l'une quelconque des revendications 1 à 3, dans laquelle la fraction protéique impure contenant PEDNF comprend un milieu conditionné d'épithélium pigmentaire rétinien.

5. Méthode selon l'une quelconque des revendications 1 à 3, dans laquelle la fraction protéique impure contenant PEDNF comprend un extrait d'un organisme non-humain transformé avec une molécule d'ADN recombinante comprenant un gène PEDNF dans une forme capable d'exprimer le gène PEDNF.

6. Méthode selon l'une quelconque des revendications 1 à 3, dans laquelle la fraction protéique impure contenant PEDNF comprend un milieu dans lequel un organisme non-humain, transformé avec une molécule d'ADN recombinante comprenant un gène PEDNF dans une forme capable d'exprimer le gène PEDNF, est cultivé.


### SEQUENCE LISTING

(1) GENERAL INFORMATION:
   (i) APPLICANT: Johnson, Lincoln V.
      Tombran-Tink, Joyce
   (ii) TITLE OF INVENTION: Retinal Pigmented Epithelium Derived Neurotrophic Factor
   (iii) NUMBER OF SEQUENCES: 1
   (iv) CORRESPONDENCE ADDRESS:
      (A) ADDRESSEE: Christie, Parker & Hale
      (B) STREET: P.O. Box 7068
      (C) CITY: Pasadena
      (D) STATE: CA
      (E) COUNTRY: USA
      (F) ZIP: 91109-7068
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US
      (B) FILING DATE:
      (C) CLASSIFICATION:
   (vii) PRIOR APPLICATION DATA: NONE
   (viii) ATTORNEY/AGENT INFORMATION:
      (A) NAME: Sharp Esq., Janice A.
      (B) REGISTRATION NUMBER: 34,051
      (C) REFERENCE/DOCKET NUMBER: U66:23860
   (ix) TELECOMMUNICATION INFORMATION:
      (A) TELEPHONE: (818)795-5843
      (B) TELEFAX: (818)577-1769
(2) INFORMATION FOR SEQ ID NO:1:
   (i) SEQUENCE CHARACTERISTICS:
      (A) LENGTH: 1503 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Homo sapiens
      (D) DEVELOPMENTAL STAGE: Fetus
      (F) TISSUE TYPE: Eye
   (vii) IMMEDIATE SOURCE:
      (A) LIBRARY: Charon BS Library of A. Swaroop
      (B) CLONE: PEDNF
   (ix) FEATURE:
      (A) NAME/KEY: prim_transcript
      (B) LOCATION: 117..1370
   (ix) FEATURE:
      (A) NAME/KEY: CDS
      (B) LOCATION: 117..1370
   (x) PUBLICATION INFORMATION:
      (A) AUTHORS: Steele, Fintan R.
         Chader, Gerald J.
         Johnson, Lincoln V.
         Tombran-Tink, Joyce
      (B) TITLE: Pigmented Epithelium-Derived Factor (PEDNF):
         Neurotrophic Activity and Identification as a
         Unique Member of the Serine Protease Inhibitor
         (SERPIN) Gene Family.
      (C) JOURNAL: Proc. Natl Acad. Sci. USA
      (G) DATE: 1993
      (K) RELEVANT RESIDUES IN SEQ ID NO:1: FROM 1 TO 1502
   (xi) SEQUENCE DESCRIPTION: SEQ ID NO:1:
   GGACGCTGGA TTAGAAGGCA GCAAAAAAAG ATCTGTGCTG GCTGGAGCCC CCTCAGTGTG 60
   CAGGCTTAGA GGGACTAGGC TGGGTGTGGA GCTGCAGCGT ATCCACAGGC CCCAGG     116
   ATG CAG GCC CTG GTG CTA CTC CTC TGC ATT GGA GCC CTC CTC GGG CAC   164
   AGC AGC TGC CAG AAC CCT GCC AGC CCC CCG GAG GAG GGC TCC CCA GAC   212
   CCC GAC AGC ACA GGG GCG CTG GTG GAG GAG GAG GAT CCT TTC TTC AAA   260
   GTC CCC GTG AAC AAG CTG GCA GCG GCT GTC TCC AAC TTC GGC TAT GAC   308
   CTG TAC CGG GTG CGA TCC AGC ATG AGC CCC ACG ACC AAC GTG CTC CTG   356
   TCT CCT CTC AGT GTG GCC ACG GCC CTC TCG GCC CTC TCG CTG GGA GCG   404
   GAC GAG CGA ACA GAA TCC ATC ATT CAC CGG GCT CTC TAC TAT GAC TTG 452
   ATC AGC AGC CCA GAC ATC CAT GGT ACC TAT AAG GAG CTC CTT GAC ACG 500
   GTC ACT GCC CCC CAG AAG AAC CTC AAG AGT GCC TCC CGG ATC GTC TTT 548
   GAG AAG AAG CTG CGC ATA AAA TCC AGC TTT GTG GCA CCT CTG GAA AAG 596
   TCA TAT GGG ACC AGG CCC AGA GTC CTG ACG GGC AAC CCT CGC TTG GAC 644
   CTG CAA GAG ATC AAC AAC TGG GTG CAG GCG CAG ATG AAA GGG AAG CTC 692
   GCC AGG TCC ACA AAG GAA ATT CCC GAT GAG ATC AGC ATT CTC CTT CTC 740
   GGT GTG GCG CAC TTC AAG GGG CAG TGG GTA ACA AAG TTT GAC TCC AGA 788
   AAG ACT TCC CTC GAG GAT TTC TAC TTG GAT GAA GAG AGG ACC GTG AGG 836
   GTC CCC ATG ATG TCG GAC CCT AAG GCT GTT TTA CGC TAT GGC TTG GAT 884
   TCA GAT CTC AGC TGC AAG ATT GCC CAG CTG CCC TTG ACC GGA AGC ATG 932
   AGT ATC ATC TTC TTC CTG CCC CTG AAA GTG ACC CAG AAT TTG ACC TTG 980
   ATA GAG GAG AGC CTC ACC TCC GAG TTC ATT CAT GAC ATA GAC CGA GAA   1028
   CTG AAG ACC GTG CAG GCG GTC CTC ACT GTC CCC AAG CTG AAG CTG AGT   1076
   TAC GAA GGC GAA GTC ACC AAG TCC CTG CAG GAG ATG AAG CTG CAA TCC   1124
   TTG TTT GAT TCA CCA GAC TTT AGC AAG ATC ACA GGC AAA CCC ATC AAG   1172
   CTG ACT CAG GTG GAA CAC CGG GCT GGC TTT GAG TGG AAC GAG GAT GGG   1220
   GCG GGA ACC ACC CCC AGC CCA GGG CTG CAG CCT GCC CAC CTC ACC TTC   1268
   CCG CTG GAC TAT CAC CTT AAC CAG CCT TTC ATC TTC GTA CTG AGG GAC   1316
   ACA GAC ACA GGG GCC CTT CTC TTC ATT GGC AAG ATT CTG GAC CCC AGG   1364
   GGC CCC TAATATCCCA GTTTAATATT CCAATACCCT AGAAGAAAAC CCGAGGGACA    1420
   GCAGATTCCA CAGGACACGA AGGCTGCCCC TGTAAGGTTT CAATGCATAC AATAAAAGAG 1480
   CTTTATCCCT AAAAAAAAAA AAA                                         1503